# EUROPEAN PATENT APPLICATION

(11) **EP 2 311 810 A1**
(43) Date of publication of application: **20.04.2011**
(21) Application number: 09794531.5
(22) Date of filing: 10.07.2009
(51) Int. Cl.: C07D 217/06, A61K 31/4545, A61K 31/4725, A61K 31/55, A61M 1/36, A61P 7/04, C07D 401/12, C07D 401/14

(54) **AMIDINE DERIVATIVE**

(30) Priority: 11.07.2008 JP 2008181882
(71) Applicant: Ajinomoto Co., Inc., Tokyo 104-8315 (JP)
(72) Inventor: MATSUMOTO, Kayo, Kawasaki-shi Kanagawa 210-8681 (JP); SUGIKI, Masayuki, Kawasaki-shi Kanagawa 210-8681 (JP); TAKAYANAGI, Masaru, Kawasaki-shi Kanagawa 210-8681 (JP); NOGI, Yasuko, Kawasaki-shi Kanagawa 210-8681 (JP); TANIGUCHI, Shinya, Kawasaki-shi Kanagawa 210-8681 (JP); UENO, Satoko, Kawasaki-shi Kanagawa 210-8681 (JP); SHIRAI, Yoshiaki, Kawasaki-shi Kanagawa 210-8681 (JP)
(74) Representative: Nicholls, Kathryn Margaret
(86) International application number: PCT/JP2009/062636
(87) International publication number: WO 2010/005087

(57) **Abstract**

Provision of a novel amidine derivative or a pharmaceutically acceptable salt thereof having an activated blood coagulation factor X-inhibitory activity. A compound represented by the formula (I) 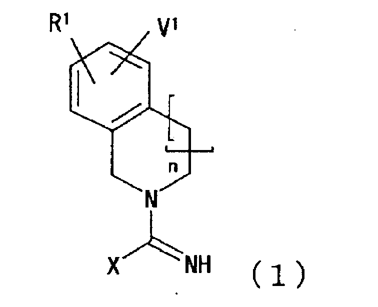 wherein each symbol is as defined above, or a pharmaceutically acceptable salt thereof.

## Description

### [Technical Field]

The present invention relates to a novel amidine derivative having an activated blood coagulation factor X (hereinafter sometimes to be abbreviated as FXa)-inhibitory activity, a production method thereof, a production intermediate therefor and a pharmaceutical composition containing the amidine derivative.
In addition, the present invention also relates to use of a low molecular weight FXa inhibitor, particularly a low molecular weight FXa inhibitor having a short half-life in blood, in a circuit for extracorporeal blood circulation, and the like.

### [Background Art]

The extracorporeal blood circulation is performed by a circulation circuit which draws the blood from the body to the outside using an artificial channel for blood flow and resends the blood into the body via an apparatus for applying a given treatment (e.g., artificial heart lung apparatus, blood purification apparatus etc.). Blood purification therapy such as hemodialysis, blood filtration, hemodialysis filtration, plasma exchange and the like, heart-lung bypass during open-heart surgery and the like sometimes require an extracorporeal blood circulation treatment. Typical examples of the blood purification apparatus include dialyzer and the like.

When blood contacts a foreign substance, an intrinsic blood coagulation cascade is generally activated and the blood is finally coagulated to lose flowability. Since an artificial channel for blood flow used for extracorporeal blood circulation and a circuit for extracorporeal blood circulation including various apparatuses are foreign substances, the blood coagulates on contact therewith. Thus, a treatment by some method to prevent blood coagulation in the circuit for extracorporeal blood circulation is necessary.

Conventionally, in an attempt to prevent blood coagulation in the circuit for extracorporeal blood circulation, anti(blood)coagulation drugs(agents) (anticoagulants) such as unfractionated heparin, low molecular weight heparin and the like are used.

However, unfractionated heparin is known to have a risk of bleeding tendency since it has a thrombin inhibitory activity in addition to an FXa inhibitory activity, and therefore, cannot be used for patients with a high bleeding risk. In addition, low molecular weight heparin is a medicament obtained by chemically treating heparin and inhibits FXa more selectively than thrombin. Since it does not have a thrombin inhibitory activity, the risk of bleeding tendency decreases, and therefore, is used for patients with a bleeding tendency. On the other hand, when a bleeding symptom is developed, hemostasis is difficult since low molecular weight heparin has a long elimination half-life.

Some serine protease inhibitors also have an anticoagulant action and, for example, nafamostat mesilate is used for some kind of extracorporeal blood circulation such as hemodialysis and the like. Since nafamostat mesilate has a short elimination half-life in the body, it is used for patients already having a bleeding lesion. However, the inhibitory activity of nafamostat mesilate against FXa and thrombin is not strong, and the anticoagulant effect thereof is weak.

As mentioned above, all medicaments still have problems to be solved, and a more effective and safer medicament is desired.

Note that patients linked to an extracorporeal circulation circuit face the problem of blood coagulation only when using the circuit, and are often different from patients requiring continued prevention of blood coagulation. Conventionally, it has not been expected at all that a selective low molecular weight FXa inhibitor having a short half-life in blood can be used safely and conveniently as an anti(blood)coagulation drug(agent) for prevention of blood coagulation in a circuit for extracorporeal blood circulation, and requires apparently lower level of treatment for and attention to hemostasis after completion of extracorporeal blood circulation.

As an amidine compound exhibiting an anticoagulant activity based on an FXa selective inhibitory action, moreover, compounds described in patent documents 1 and 2 are known. However, the compounds are clearly different in structure from the compound of the present invention.

### [Document List]

### [Patent Documents]

patent document 1: WO98/31661
patent document 2: WO99/64392

### [Summary of the Invention]

### [Problems to be Solved by the Invention]

The present invention aims to provide a novel amidine derivative or a pharmaceutically acceptable salt thereof.

The present invention aims to provide a production method of the above-mentioned amidine derivative or a pharmaceutically acceptable salt thereof, and a production intermediate therefor.

The present invention also aims to provide an activated blood coagulation factor X inhibitor comprising the above-mentioned amidine derivative or a pharmaceutically acceptable salt thereof.

The present invention also aims to provide an anti(blood)coagulation drug(agent) comprising the above-mentioned amidine derivative or a pharmaceutically acceptable salt thereof.

The present invention also aims to provide a pharmaceutical composition comprising the above-mentioned amidine derivative or a pharmaceutically acceptable salt thereof.

The present invention also aims to provide a novel anti(blood)coagulation drug(agent) or pharmaceutical composition for a circuit for extracorporeal blood circulation.

The present invention also aims to provide a novel method of preventing thrombus formation in a circuit for extracorporeal blood circulation.

### [Means of Solving the Problems]

In view of the aforementioned situation, the present inventors have conducted various studies and found that a particular novel amidine derivative having an ester bond in a molecule, a compound represented by A'-COO-B', wherein A' and B' are each an organic group and at least one of them contains an amidino group or a guanidino group structure, has a superior activated blood coagulation factor X-inhibitory activity, has a short half-life in blood and is useful as an anti(blood)coagulation drug(agent) for a circuit for extracorporeal blood circulation, which resulted in the completion of the present invention.

Accordingly, the present invention is as follows.
[1] An amidine derivative represented by the following formula (1):

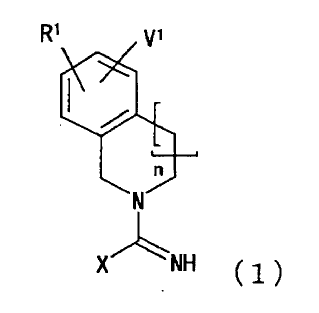

wherein,
X is a C₁₋₆ alkyl group or an amino group,
V¹ is a hydrogen atom, a hydroxyl group, a halogen atom, a C₁₋₁₀ alkyl group optionally having substituent(s), a C₁₋₁₀ alkoxy group optionally having substituent(s), a C₁₋₁₀ alkylamino group optionally having substituent(s), an aliphatic nitrogen-containing heterocyclic group having 2 to 8 carbon atoms, a C₁₋₁₀ alkylthio group optionally having substituent(s), a cyano group, a nitro group, a carboxyl group, a carbamoyl group optionally having substituent(s) or a C₂₋₁₀ alkoxycarbonyl group optionally having substituent(s),
n is an integer of 0 to 2, and
R¹ is a group represented by the following formula (2-1) or (2-2):

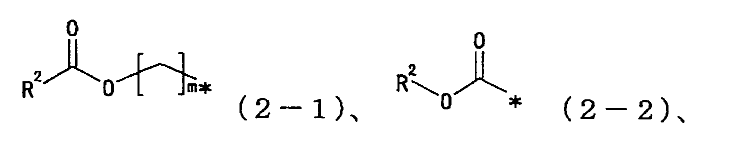

wherein,
m is an integer of 0 to 2, and
R² is a group represented by the following formula (3):

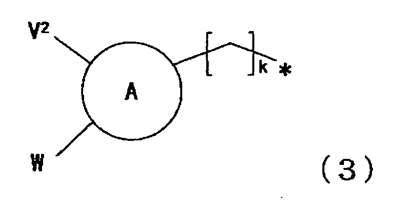

wherein,
k is an integer of 0 to 2,
ring A is a C₆₋₁₀ aryl group, a C₁₋₁₀ heteroaryl group, an aliphatic nitrogen-containing heterocyclic group having 2 to 8 carbon atoms or a C₃₋₁₀ cycloalkyl group,
V² is a hydrogen atom, a hydroxyl group, a halogen atom, a C₁₋₁₀ alkyl group optionally having substituent(s), a C₁₋₁₀ alkoxy group optionally having substituent(s), a C₁₋₁₀ alkylamino group optionally having substituent(s), an aliphatic nitrogen-containing heterocyclic group having 2 to 8 carbon atoms, a C₁₋₁₀ alkylthio group optionally having substituent(s), a cyano group, a nitro group, a carboxyl group, a carbamoyl group optionally having substituent(s) or a C₂₋₁₀ alkoxycarbonyl group optionally having substituent(s), and
W is an amidino group optionally substituted by C₁₋₆ alkyl group(s), a guanidino group optionally substituted by C₁₋₆ alkyl group(s), a C₁₋₆ alkyl group optionally having an imino group at the 1-position or a group represented by the following formula (4):

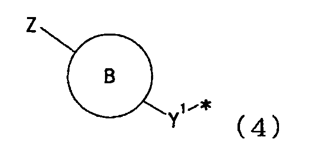

wherein,
ring B is a C₁₋₁₀ heteroaryl group, or an aliphatic nitrogen-containing heterocyclic group having 2 to 8 carbon atoms,
Y¹ is a single bond, -NH- optionally substituted by a C₁₋₆ alkyl group, an oxygen atom, a sulfur atom, a methylene group or -CO-, and
Z is a hydrogen atom, a halogen atom, an amidino group optionally substituted by C₁₋₆ alkyl group(s), a guanidino group optionally substituted by C₁₋₆ alkyl group(s), or a C₁₋₆ alkyl group optionally having an imino group at the 1-position, or a pharmaceutically acceptable salt thereof.
[2] The amidine derivative of the above-mentioned [1], which is represented by the following formula (1-2):

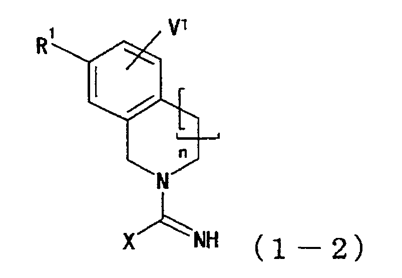

wherein R¹, V¹, X and n are as defined in the above-mentioned [1],
or a pharmaceutically acceptable salt thereof.
[3] The amidine derivative of the above-mentioned [2], wherein, in the formula (3),
ring A is a phenyl group, a pyridyl group, a thiophenyl group, a piperidyl group or a piperazinyl group, and
V² is a hydrogen atom, a halogen atom, a C₁₋₆ alkyl group, a carboxyl group, a C₁₋₆ alkoxy group optionally having substituent(s) or a C₂₋₁₀ alkoxycarbonyl group optionally having substituent(s),
or a pharmaceutically acceptable salt thereof.
[4] The amidine derivative of the above-mentioned [3], wherein, in the formula (3),
W is a group represented by the formula (4),
ring B is an aliphatic nitrogen-containing heterocyclic group having 2 to 8 carbon atoms,
Y¹ is an oxygen atom, a sulfur atom or a methylene group, and
Z is a hydrogen atom, a halogen atom, an amidino group or a C₁₋₆ alkyl group optionally having an imino group at the 1-position, or a pharmaceutically acceptable salt thereof.
[5] The amidine derivative of the above-mentioned [3], wherein, in the formula (3),
W is a group represented by the formula (4),
ring B is a pyridyl group, and
Y¹ is a single bond,
or a pharmaceutically acceptable salt thereof.
[6] An amidine derivative represented by the following formula (5):

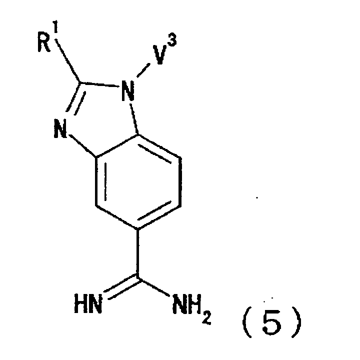

wherein,
V³ is a hydrogen atom or a group represented by the following formula (6):

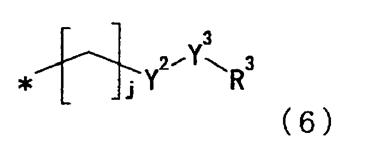

wherein,
R³ is a hydrogen atom, a C₁₋₆ alkyl group optionally having substituent(s), a C₃₋₁₀ cycloalkyl group optionally having substituent(s), a carboxyl group, a C₂₋₇ alkoxycarbonyl group, a C₆₋₁₀ aryl group optionally having substituent(s), a heteroaryl group optionally having substituent(s) or a saturated aliphatic nitrogen-containing heterocyclic group having 2 to 8 carbon atoms,
Y² is an oxygen atom, -CO-, -CO₂-, -SO₂-, -CONH- or - CH=CH-,
Y³ is -(CH₂)ᵢ- or -(CH₂)_{i'}-CUU'-(CH₂)_{i"}- (wherein U and U' are the same or different and each is a hydrogen atom or a C₁₋₆ alkyl group, and i, i' and i" are each independently an integer of 0 to 3), and
j is an integer of 0 to 3,
R¹ is as defined in the above-mentioned [1],
or a pharmaceutically acceptable salt thereof.
[7] The amidine derivative of the above-mentioned [6], wherein, in the formula (5),
V³ is a group represented by the formula (6), and in the formula (3),
ring A is a phenyl group, a pyridyl group, a thiophenyl group, a piperidyl group or a piperazinyl group, and
V² is a hydrogen atom, a halogen atom, a C₁₋₆ alkyl group, a carboxyl group, a C₁₋₆ alkoxy group optionally having substituent(s) or a C₂₋₁₀ alkoxycarbonyl group optionally having substituent(s),
or a pharmaceutically acceptable salt thereof.
[8] The amidine derivative of the above-mentioned [7], wherein, in the formula (5),
V³ is a group represented by the formula (6), and in the formula (3),
W is a group represented by the formula (4),
ring B is an aliphatic nitrogen-containing heterocyclic group having 2 to 8 carbon atoms,
Y¹ is an oxygen atom, a sulfur atom or a methylene group, and
Z is a hydrogen atom, an amidino group or a C₁₋₆ alkyl group optionally having an imino group at the 1-position,
or a pharmaceutically acceptable salt thereof.
[9] The amidine derivative of the above-mentioned [7], wherein, in the formula (5),
V³ is a group represented by the formula (6), and
in the formula (3),
W is a group represented by the formula (4),
ring B is a pyridyl group, and
Y¹ is a single bond,
or a pharmaceutically acceptable salt thereof.
[10] An activated blood coagulation factor X inhibitor comprising the amidine derivative of any of the above-mentioned [1] to [9], or a pharmaceutically acceptable salt thereof.
[11] A pharmaceutical composition comprising the amidine derivative of any of the above-mentioned [1] to [9], or a pharmaceutically acceptable salt thereof.
[12] The pharmaceutical composition of the above-mentioned
[11], which is an anti-blood coagulation drug.
[13] The pharmaceutical composition of the above-mentioned
[12], which is an anti-blood coagulation drug for a circuit for extracorporeal blood circulation.
[14] The pharmaceutical composition of the above-mentioned
[12], which is an anti-blood coagulation drug for hemodialysis.
[15] A dialysis solution or dialysis concentrate comprising the amidine derivative of any of the above-mentioned [1] to [9], or a pharmaceutically acceptable salt thereof.
[16] An anti-blood coagulation drug for a circuit for extracorporeal blood circulation, comprising a low molecular weight FXa inhibitor as an active ingredient.
[17] The anti-blood coagulation drug of the above-mentioned [16], wherein the low molecular weight FXa inhibitor is rapidly cleared from the blood.
[18] The anti-blood coagulation drug of the above-mentioned [17], wherein the low molecular weight FXa inhibitor is a selective FXa inhibitor.
[19] A method of inhibiting blood coagulation in a mammal, comprising administering an effective amount of the amidine derivative of any of the above-mentioned [1] to [9], or a pharmaceutically acceptable salt thereof to the mammal.

The present invention also provides an activated blood coagulation factor X inhibitor, anti(blood)coagulation drug(agent) or pharmaceutical composition comprising the above-mentioned amidine derivative or a pharmaceutically acceptable salt thereof.

The present invention also provides an anti(blood)coagulation drug(agent) for a circuit for extracorporeal blood circulation, comprising a low molecular weight FXa inhibitor as an active ingredient.

The present invention also provides a method of preventing thrombus formation in a circuit for extracorporeal blood circulation, comprising incorporating a low molecular weight FXa inhibitor into a constituent element of the circuit for extracorporeal blood circulation.

### [Description of Embodiments]

The terms used in the present specification are defined in the following.
The "aryl group" is a monocyclic - bicyclic aromatic hydrocarbon ring group or phenyl group condensed with a 5- to 8-membered cycloalkyl ring, which may have substituent(s). Examples of the "aryl group" include a phenyl group, a naphthyl group, an indanyl group and a tetrahydronaphthalenyl group. Generally, it has 6 to 14 carbon atoms, preferably 6 to 10 carbon atoms, a phenyl group or a naphthyl group is more preferable, and a phenyl group is particularly preferable. The "C₆₋₁₄ aryl group" is the above-mentioned aryl group having 6 to 14 carbon atoms, and the "C₆₋₁₀ aryl group" is the above-mentioned aryl group having 6 to 10 carbon atoms.

The "heteroaryl group" is a 5- to 10-membered monocyclic or bicyclic aromatic heterocyclic group containing, as ring atom, 1 to 6 hetero atoms selected from an oxygen atom, a sulfur atom and a nitrogen atom. Examples of the "heteroaryl group" include a pyridyl group, a pyridazinyl group, a pyrimidinyl group, a pyrazinyl group, a furyl group, a thiophenyl group, a pyrrolyl group, an isoxazolyl group, an oxazolyl group, an isothiazolyl group, a thiazolyl group, a pyrazolyl group, an imidazolyl group, an oxadiazolyl group, a thiadiazolyl group, a triazolyl group, a tetrazolyl group, a benzofuryl group, a benzothiophenyl group, an indolyl group, an isoindolyl group, a benzoxazolyl group, a benzothiazolyl group, a benzimidazolyl group, an indazolyl group, a benzisoxazolyl group, a benzisothiazolyl group, a benzofurazanyl group, a benzothiadiazolyl group, a purinyl group, a quinolinyl group, an isoquinolinyl group, a cinnolinyl group, a phthalazinyl group, a quinazolinyl group, a quinoxalinyl group, a pteridinyl group, an imidazooxazolyl group, an imidazothiazolyl group, an imidazoimidazolyl group and the like. A heteroaryl group having 1 to 10 carbon atoms is preferable, a pyridyl group, a pyridazinyl group, a pyrimidinyl group, a pyrazinyl group, a furyl group, a thiophenyl group, a pyrrolyl group, an isoxazolyl group, an oxazolyl group, an isothiazolyl group, a thiazolyl group, a pyrazolyl group, an imidazolyl group, an oxadiazolyl group, a thiadiazolyl group, a triazolyl group and a tetrazolyl group are more preferable, and a pyridyl group and a thiophenyl group are further preferable. The "C₁₋₁₀ heteroaryl group" is the above-mentioned "heteroaryl group" having 1 to 10 carbon atoms, and the "C₁₋₉ heteroaryl group" is the above-mentioned heteroaryl group having 1 to 9 carbon atoms. The "nitrogen-containing heteroaryl group having 1 to 10 carbon atoms" is the above-mentioned "C₁₋₁₀ heteroaryl group" having at least one nitrogen atom as a ring atom.

The "aliphatic nitrogen-containing heterocyclic group" (i.e., a nonaromatic nitrogen-containing heterocyclic group) is a 4- to 10-membered monocyclic or bicyclic saturated or partly unsaturated aliphatic heterocyclic group having at least one nitrogen atom and further optionally having one or more oxygen atoms or sulfur atoms as a ring atom. Examples of the "aliphatic nitrogen-containing heterocyclic group" include a pyrrolidinyl group, a pyrazolidinyl group, an imidazolidinyl group, a pyrrolinyl group, a pyrazolinyl group, an imidazolyl group, a thiazolidinyl group, a piperidyl group, a piperidino group, a piperazinyl group, a quinuclidinyl group, a morpholino group, a morpholinyl group, a thiomorpholino group, a thiomorpholinyl group, a homopiperidyl group, a homopiperazinyl group, an indolinyl group, an isoindolinyl group, a tetrahydroquinolinyl group, a tetrahydroisoquinolinyl group and the like, preferably, a pyrrolidinyl group, a piperidyl group, a piperazinyl group, a tetrahydroquinolinyl group, and a tetrahydroisoquinolinyl group. An aliphatic nitrogen-containing heterocyclic group having 2 to 8 carbon atoms is preferable, and a piperidyl group, a piperazinyl group and a pyrrolidinyl group are particularly preferable. The "aliphatic nitrogen-containing heterocyclic group having 2 to 8 carbon atoms" is the above-mentioned "aliphatic nitrogen-containing heterocyclic group" having 2 to 8 carbon atoms. The "aliphatic nitrogen-containing heterocyclic group having 1 to 9 carbon atoms" is the above-mentioned "aliphatic nitrogen-containing heterocyclic group" having 1 to 9 carbon atoms. The "saturated aliphatic nitrogen-containing heterocyclic group having 2 to 8 carbon atoms" is the above-mentioned "aliphatic nitrogen-containing heterocyclic group having 2 to 8 carbon atoms" which is saturated.

The "cycloalkyl group" is an aliphatic hydrocarbon ring group, which may contain a double bond in the ring. The "cycloalkyl group" preferably has 3 to 10 carbon atoms and examples thereof include a cyclopropyl group, a cyclobutyl group, a cyclopentyl group, a cyclohexyl group, a cycloheptyl group, a cyclohexenyl group, a cyclopentenyl group and the like, with particular preference given to a cyclohexyl group. The "C₃₋₁₀ cycloalkyl group" is the above-mentioned "cycloalkyl group" having 3 to 10 carbon atoms. The "C₃₋₈ cycloalkyl group" is the above-mentioned "cycloalkyl group" having 3 to 8 carbon atoms.

The "alkyl group" or "alkyl group moiety" in the "alkylthio group", "alkylamino group", "alkoxy group", "alkoxycarbonyl group" and the like is a straight chain, branched chain, cyclic or partly cyclic aliphatic hydrocarbon group, and examples thereof include a methyl group, an ethyl group, a propyl group, an isopropyl group, a butyl group, an isobutyl group, a sec-butyl group, a tert-butyl group, a cyclopropylmethyl group, a cyclobutyl group, a pentyl group, an isopentyl group, a neopentyl group, a hexyl group, a heptyl group, an octyl group, a nonyl group, a decyl group, a 1,1-dimethyl-propyl group, a cyclopropyl group, a cyclopentyl group, a cyclohexyl group, a cycloheptyl group, a cyclooctyl group and the like. The alkyl group moiety preferably has 1 to 10 carbon atoms, more preferably 1 to 6 carbon atoms, further preferably 1 to 3 carbon atoms. Particularly preferred are a methyl group, an ethyl group, an isopropyl group, an isobutyl group and a cyclopropyl group, and more preferred are a methyl group, an ethyl group, an isopropyl group and a cyclopropyl group.

The "C₁₋₁₀ alkyl group" is the above-mentioned "alkyl group" having 1 to 10 carbon atoms, and the "C₁₋₆ alkyl group" is the above-mentioned "alkyl group" having 1 to 6 carbon atoms.

The "C₁₋₁₀ alkylthio group" has an alkyl group moiety which is the above-mentioned "alkyl group moiety" having 1 to 10 carbon atoms. Specific examples thereof include a methylthio group, an ethylthio group, a propylthio group, an isopropylthio group, a butylthio group, an isobutylthio group, a sec-butylthio group, a tert-butylthio group, a cyclopropylmethylthio group, a pentylthio group, an isopentylthio group, a neopentylthio group, a hexylthio group, a heptylthio group, an octylthio group, a nonylthio group, a decylthio group, a 1,1-dimethyl-propylthio group, a cyclopropylthio group, a cyclobutylthio group, a cyclopentylthio group, a cyclohexylthio group, a cycloheptylthio group, a cyclooctylthio group and the like.
The "C₁₋₆ alkylthio group" is one having 1 to 6 carbon atoms from among the above-mentioned "C₁₋₁₀ alkylthio group".

The "C₁₋₁₀ alkylamino group" is an amino group mono- or di-substituted by the above-mentioned "alkyl group moiety" having 1 to 10 carbon atoms. Specific examples thereof include mono(alkyl)amino groups such as a methylamino group, an ethylamino group, a propylamino group, an isopropylamino group, a butylamino group, an isobutylamino group, a sec-butylamino group, a tert-butylamino group, a cyclopropylmethylamino group, a pentylamino group, an isopentylamino group, a neopentylamino group, a hexylamino group, a heptylamino group, an octylamino group, a nonylamino group, a decylamino group, a (1,1-dimethyl-propyl)amino group, a cyclopropylamino group, a cyclobutylamino group, a cyclopentylamino group, a cyclohexylamino group, a cycloheptylamino group, a cyclooctylamino group and the like; and di(alkyl)amino groups such as a dimethylamino group, a diethylamino group, a dipropylamino group, a diisopropylamino group, a dibutylamino group, a diisobutylamino group, a di-sec-butylamino group, a di-tert-butylamino group, a di(cyclopropylmethyl)amino group, a dipentylamino group, a diisopentylamino group, a dineopentylamino group, a dihexylamino group, an N-methyl-N-ethylamino group, an N-methyl-N-propylamino group, an N-methyl-N-isopropylamino group, an N-methyl-N-butylamino group, an N-methyl-N-isobutylamino group, an N-methyl-N-sec-butylamino group, an N-methyl-N-tert-butylamino group, an N-ethyl-N-propylamino group, an N-ethyl-N-isopropylamino group, an N-ethyl-N-butylamino group, an N-ethyl-N-isobutylamino group, an N-ethyl-N-sec-butylamino group, an N-ethyl-N-tert-butylamino group and the like. The "alkylamino group having 1 to 6 carbon atoms" is the above-mentioned "C₁₋₁₀ alkylamino group" wherein 1 or 2 alkyl group moieties each have 1 to 6 carbon atoms.

The "C₁₋₁₀ alkoxy group" has an alkyl group moiety which is the above-mentioned "alkyl group moiety" having 1 to 10 carbon atoms. Specific examples thereof include a methoxy group, an ethoxy group, a propoxy group, an isopropoxy group, a butoxy group, an isobutoxy group, a sec-butoxy group, a tert-butoxy group, a cyclopropylmethoxy group, a pentyloxy group, an isopentyloxy group, a neopentyloxy group, a hexyloxy group, a heptyloxy group, an octyloxy group, a nonyloxy group, a decyloxy group, a 1,1-dimethyl-propoxy group, a cyclopropoxy group, a cyclobutoxy group, a cyclopentyloxy group, a cyclohexyloxy group, a cycloheptyloxy group, a cyclooctyloxy group and the like. The "C₁₋₆ alkoxy group" is the above-mentioned "C₁₋₁₀ alkoxy group" having 1 to 6 carbon atoms.

The "C₂₋₁₀ alkoxycarbonyl group" has an alkyl group moiety which is the above-mentioned "alkyl group moiety" having 1 to 9 carbon atoms. Specific examples thereof include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a cyclopropylmethoxycarbonyl group, a pentyloxycarbonyl group, an isopentyloxycarbonyl group, a neopentyloxycarbonyl group, a hexyloxycarbonyl group, a heptyloxycarbonyl group, an octyloxycarbonyl group, a nonyloxycarbonyl group, a (1,1-dimethyl-propoxy)carbonyl group, a cyclopropoxycarbonyl group, a cyclobutoxycarbonyl group, a cyclopentyloxycarbonyl group, a cyclohexyloxycarbonyl group, a cycloheptyloxycarbonyl group, a cyclooctyloxycarbonyl group and the like. The "C₂₋₇ alkoxycarbonyl group" is the above-mentioned "C₂₋₁₀ alkoxycarbonyl group" having 2 to 7 carbon atoms.

Examples of the "halogen atom" include a fluorine atom, a chlorine atom, a bromine atom, an iodine atom and the like, with preference given to a fluorine atom and a chlorine atom.

The "alkylamino group" or "alkylamino moiety" as a component of "carbamoyl group substituted by alkyl group(s)" (when the substituent of the "carbamoyl group optionally having substituent(s)" is alkyl group), "amidino group substituted by (C₁₋₆)alkyl group(s)", "guanidino group substituted by (C₁₋₆)alkyl group(s)" and the like also includes a monoalkylamino group and a dialkylamino group. In the dialkylamino group, two alkyl groups may be the same or different, and may be bonded to each other to form a ring (e.g., nitrogen-containing heterocycle (e.g., a pyrrolidine ring, a pyrroline ring) corresponding to the above-mentioned "nitrogen-containing heterocyclic group" etc.).

In the present specification, examples of the substituent of "optionally having substituent(s)" include
(1) a halogen atom,
(2) a hydroxyl group,
(3) an amino group,
(4) a C₁₋₁₀ alkyl group, preferably a C₁₋₆ alkyl group,
(5) a C₂₋₁₀ alkenyl group, preferably C₂₋₆ alkenyl group (e.g., a vinyl group, an allyl group, an isopropenyl group, a 1-butenyl group, a 2-butenyl group, a 3-butenyl group, a butadienyl group, a 2-methylallyl group, a hexatrienyl group, a 3-octenyl group etc.),
(6) a C₂₋₁₀ alkynyl group, preferably C₂₋₆ alkynyl group (e.g., an ethynyl group, a 2-propynyl group, an isopropynyl group, a butynyl group, a tert-butynyl group, a 3-hexynyl group etc.),
(7) a C₁₋₆ alkoxy group optionally substituted by phenyl,
(8) a C₁₋₆ alkylamino group,
(9) a cyano group,
(10) a guanidino group,
(11) a carboxyl group,
(12) a carbamoyl group,
(13) a C₆₋₁₄ aryl group, preferably a C₆₋₁₀ aryl group,
(14) a C₁₋₁₀ heteroaryl group, preferably a C₁₋₉ heteroaryl group,
(15) a C₃₋₁₀ cycloalkyl group, preferably a C₃₋₈ cycloalkyl group,
(16) an aliphatic nitrogen-containing heterocyclic group having 1 to 9 carbon atoms, preferably an aliphatic nitrogen-containing heterocyclic group having 2 to 8 carbon atoms,
(17) a C₁₋₁₀ alkylthio group, preferably a C₁₋₆ alkylthio group,
(18) a C₁₋₁₀ acyloxy group, preferably a C₁₋₆ acyloxy group,
(19) a C₁₋₁₀ acylamino group, preferably a C₁₋₆ acylamino group,
(20) a C₁₋₁₀ alkylsulfonamide group, preferably a C₁₋₆ alkylsulfonamide group (e.g., a methylsulfonamide group, an ethylsulfonamide group, a propylsulfonamide group, an isopropylsulfonamide group, a butylsulfonamide group, an isobutylsulfonamide group, a sec-butylsulfonamide group, a tert-butylsulfonamide group, a cyclopropylmethylsulfonamide group, a pentylsulfonamide group, an isopentylsulfonamide group, a neopentylsulfonamide group, a hexylsulfonamide group, a heptylsulfonamide group, an octylsulfonamide group, a nonylsulfonamide group, a decylsulfonamide group, a (1,1-dimethyl-propyl)sulfonamide group, a cyclopropylsulfonamide group, a cyclobutylsulfonamide group, a cyclopentylsulfonamide group, a cyclohexylsulfonamide group, a cycloheptylsulfonamide group, a cyclooctylsulfonamide group etc.),
(21) a C₂₋₁₀ alkoxycarbonyl group, preferably a C₂₋₇ alkoxycarbonyl group,
   and the like.

Examples of the "acyl group moiety" as a component of "acyloxy group", "acylamino group" and the like include C₁₋₁₁ acyl groups such as a formyl group, C₂₋₁₀ alkylcarbonyl groups (e.g., an acetyl group, an ethylcarbonyl group, a propylcarbonyl group, an isopropylcarbonyl group, a butylcarbonyl group, an isobutylcarbonyl group, a sec-butylcarbonyl group, a tert-butylcarbonyl group, a cyclopropylmethylcarbonyl group, a pentylcarbonyl group, an isopentylcarbonyl group, a neopentylcarbonyl group, a hexylcarbonyl group, a heptylcarbonyl group, an octylcarbonyl group, a nonylcarbonyl group, a (1,1-dimethyl-propyl)carbonyl group, a cyclopropylcarbonyl group, a cyclobutylcarbonyl group, a cyclopentylcarbonyl group, a cyclohexylcarbonyl group, a cycloheptylcarbonyl group, a cyclooctylcarbonyl group etc.), C₂₋₁₁ arylcarbonyl groups (e.g., a benzoyl group, a 1-naphthylcarbonyl group, a 2-naphthylcarbonyl group etc.) and the like. Of these, a C₁₋₁₀ acyl group is preferable, and a C₁₋₇ acyl group is more preferable. Particularly preferred is a C₁₋₆ acyl group.

As the substituent, preferred are
(1) a halogen atom,
(2) a hydroxyl group,
(3) an amino group,
(4) a C₁₋₆ alkyl group,
(5) a C₂₋₆ alkenyl group,
(6) a C₂₋₆ alkynyl group,
(7) a C₁₋₆ alkoxy group optionally substituted by phenyl,
(8) a C₁₋₆ alkylamino group,
(9) a cyano group,
(10) a guanidino group,
(11) a carboxyl group,
(12) a carbamoyl group,
(13) a C₁₋₆ acyloxy group,
(14) a C₁₋₆ acylamino group,
(15) a C₃₋₈ cycloalkyl group,
(16) a C₁₋₆ alkylthio group,
(17) a C₁₋₆ alkylsulfonamide group, and
(18) a C₂₋₁₀ alkoxycarbonyl group.
As the substituent, more preferred is a C₁₋₆ alkoxy group (preferably, a methoxy group) optionally substituted by phenyl. Particularly preferred is a methoxy group or a benzyloxy group.
The number and position of the substituent are not particularly limited.

In addition, a compound represented by the formula (1) or (5) of the present invention (hereinafter sometimes to be abbreviated as compound (1) or (5)) includes a mixture of various stereoisomers such as geometric isomers, tautomers, optical isomers and the like, and isolated forms thereof, and stable isotope and radioactive isotope.

In the formulas (2-1), (2-2), (3), (4) and (6) in the present specification, the binding sites are shown by *.

In the formula (1),
X is preferably a methyl group or an amino group, particularly preferably an amino group,
V¹ is preferably a hydrogen atom, a lower alkoxy group (e.g., a C₁₋₄ alkoxy group) or a halogen atom, particularly preferably a hydrogen atom, and
n is preferably 0 to 2.
In the formula (2-1), m is preferably 0 or 1.

In the formula (3),
k is preferably 0 or 1,
particularly, when R¹ is a group represented by the formula (2-1), k is preferably 0, and when R¹ is a group represented by the formula (2-2), k is preferably 1, and
ring A is preferably a phenyl group, a pyridyl group, a thiophenyl group, a naphthyl group, a thienyl group, a piperidyl group or a piperazinyl group, more preferably a phenyl group, a naphthyl group, a thienyl group or a piperidyl group, and particularly preferably a phenyl group or a piperidyl group.
In another embodiment, ring A is preferably a phenyl group, a pyridyl group, a thiophenyl group, a piperidyl group or a piperazinyl group.
V² is preferably a hydrogen atom, a hydroxyl group, a halogen atom, a C₁₋₆ alkyl group optionally having substituent(s), a carboxyl group, a C₁₋₆ alkoxy group optionally having substituent(s), a carbamoyl group optionally having substituent(s) or a C₂₋₁₀ alkoxycarbonyl group optionally having substituent(s), more preferably is a hydrogen atom, a methyl group optionally having substituent(s) (preferably, a hydroxymethyl group, a carboxymethyl group, a methoxymethyl group, a benzyloxymethyl group), a fluorine atom, a chlorine atom, a hydroxyl group, a methoxy group, a carboxyl group, a methoxycarbonyl group, an ethoxycarbonyl group, or a carbamoyl group, and further preferably is a hydrogen atom or a methyl-group optionally having substituent(s) (preferably, a methoxymethyl group, a benzyloxymethyl group).
In another embodiment, V² is preferably a hydrogen atom, a halogen atom, a C₁₋₆ alkyl group, a carboxyl group, a C₁₋₆ alkoxy group optionally having substituent(s) or a C₂₋₁₀ alkoxycarbonyl group optionally having substituent(s).
W is preferably an amidino group, a guanidino group, a 1-iminoethyl group, an imino(pyrrolidin-1-yl)methyl group, an imino(pyrrolin-1-yl)methyl group or a group represented by the formula (4), and more preferably is a group represented by the formula (4).

In the formula (4),
ring B is preferably a C₁₋₉ heteroaryl group or an aliphatic nitrogen-containing heterocyclic group having 2 to 8 carbon atoms, more preferably a pyrrolidinyl group, a piperidyl group, a homopiperidyl group or a pyridyl group, and further preferably a piperidyl group, a pyridyl group or a pyrrolidinyl group. Particularly preferred is a pyridyl group.
In another embodiment, ring B is preferably an aliphatic nitrogen-containing heterocyclic group having 2 to 8 carbon atoms.
Y¹ is preferably a single bond, -CO-, an oxygen atom, a sulfur atom or a methylene group, particularly preferably a single bond, -CO- or an oxygen atom.
Z is preferably a hydrogen atom, a halogen atom, an amidino group or a C₁₋₆ alkyl group optionally having an imino group at the 1-position, more preferably a hydrogen atom, a fluorine atom, a chlorine atom, an amidino group or a C₁₋₆ alkyl group optionally having an imino group at the 1-position (preferably, a 1-iminoethyl group), and particularly preferably a hydrogen atom, an amidino group or a C₁₋₆ alkyl group optionally having an imino group at the 1-position (preferably, a 1-iminoethyl group).

In the formula (5), moreover,
R¹ are preferably the same as those mentioned above as preferable forms of R¹ in the above-mentioned formula (1), and
V³ is preferably a group represented by the formula (6).

In the formula (6),
R³ is preferably a cyclohexyl group or a phenyl group, more preferably a cyclohexyl group,
Y² is preferably -CONH-,
Y³ is preferably -(CH₂)ᵢ- or -(CH₂)_{i'}-CUU'-(CH₂)_{i"}- wherein i is preferably 0 or 1, i' is preferably 0 or 1, i" is preferably 0, U is preferably a hydrogen atom, and U' is preferably a methyl group, more preferably -(CH₂)ᵢ- wherein i is preferably 0, and
j is preferably 1.

In the present invention, a compound comprising a combination of preferable groups for each of the above-mentioned symbols is preferable.

More specifically, a compound represented by the following formula (1-2) is preferable:

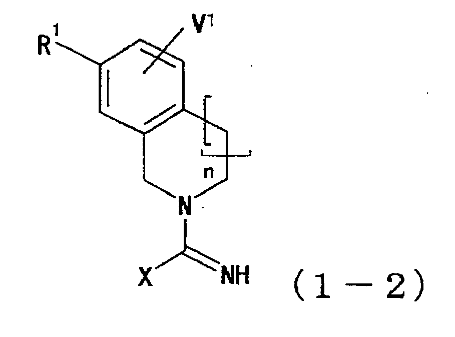

wherein R¹, V¹, X and n are as defined in the formula (1).
In the formula (1-2), preferred as R¹, V¹, X and n are the same as those mentioned above as preferable forms of R¹, V¹, X and n in the formula (1).

More specifically, though unlimitatively, the compounds described in the Examples are preferable.

In the anti(blood)coagulation drug(agent) for a circuit for extracorporeal blood circulation, comprising a low molecular weight FXa inhibitor as an active ingredient, and a method of preventing thrombus formation in a circuit for extracorporeal blood circulation, comprising incorporating a low molecular weight FXa inhibitor into a constituent element of the circuit for extracorporeal blood circulation, of the present invention, the "low molecular weight FXa inhibitor" is a compound represented by the formula (1) or the formula (5) or a pharmaceutically acceptable salt thereof, or a compound having a molecular weight of not more than 1000 and an FXa inhibitory activity, with preference given to a compound represented by the formula (1) or the formula (5). More specific examples of the compound having a molecular weight of not more than 1000 and an FXa inhibitory activity include the compounds shown in WO99/52895, WO99/10316, WO2000/59876, WO2002/28827, WO01/74791, WO96/16940, WO2002/42270 and WO2006/83003.

In addition, the above-mentioned "low molecular weight FXa inhibitor" is preferably cleared rapidly from the blood. Here, "cleared rapidly from the blood" means that the elimination half-life in the body or half-time in the stability test in plasma shown in Experimental Example 4 to be mentioned later is not more than 10 min, preferably not more than 5 min. The above-mentioned "low molecular weight FXa inhibitor" is preferably FXa-selective, and more specifically, it preferably shows a large difference between pIC₅₀(FXa) and pIC₅₀(IIa) in the inhibitory activity evaluation system shown in Experimental Examples 1 and 2 to be mentioned below.

The "extracorporeal blood circulation" is an artificial blood circulation through a blood circuit formed outside the body.
The "circuit for extracorporeal blood circulation" is a blood circuit for extracorporeal blood circulation and, for example, a blood circuit produced for connecting the body and an artificial organ when using the artificial organ. More specifically, for example, those used for artificial heart lung and hemodialysis can be mentioned. In the present invention, particularly preferred is a circuit for extracorporeal blood circulation for hemodialysis.

Representative production methods of the compounds (1) and (5) of the present invention are explained in the following.

In the formula (1), when R¹ is a group represented by the formula (2-1), intermediate (7) can be obtained by the method shown below.

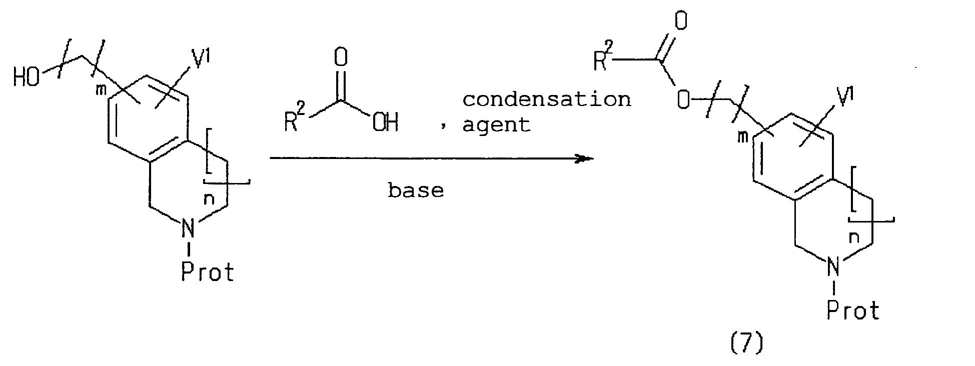

wherein Prot is an amino-protecting group (e.g., a tert-butoxycarbonyl group, a 1-methylcyclobutoxycarbonyl group and the like), and other symbols are as defined above.
For example, intermediate (7) can be obtained by reacting a nitrogen-containing heterocycle having a hydroxyl group, wherein the nitrogen is protected by a suitable protecting group (Prot) that can be removed under acidic conditions, such as tert-butyl 7-hydroxy-3,4-dihydroisoquinoline-2(1H)-carboxylate and the like, and the corresponding carboxylic acid derivative with a condensation agent such as N,N'-dicyclohexylcarbodiimide (hereinafter DCC), O-(7-azabenzotriazol-1-yl)-1,1,3,3-tetramethyluronium hexafluorophosphate (hereinafter HATU), 1-hydroxy-7-azabenzotriazole (hereinafter HOAt) and the like in, for example, a solvent such as dichloromethane, N,N'dimethylformamide (hereinafter DMF), pyridine and the like in the presence of, where necessary, an organic base such as triethylamine and 4-dimethylaminopyridine (hereinafter DMAP).

In the formula (1), when R¹ is a group represented by the formula (2-1) and m is 1, alcohol derivative (10) as an intermediate can be obtained by the method shown below.

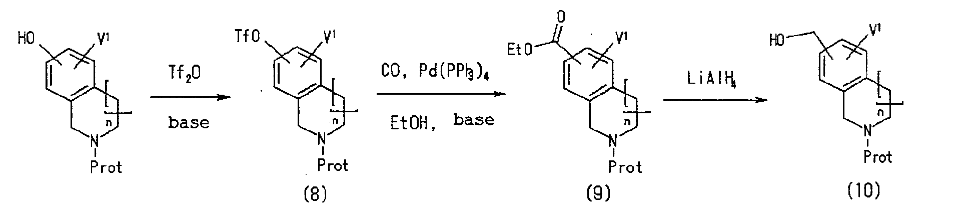

wherein each symbol is as defined above.
For example, intermediate (8) can be obtained by dissolving a nitrogen-containing heterocycle having a hydroxyl group, wherein the nitrogen is protected by a suitable protecting group (Prot) that can be removed under acidic conditions, such as tert-butyl 7-hydroxy-3,4-dihydroisoquinoline-2(1H)-carboxylate and the like, in a solvent such as dichloromethane and the like, and reacting the heterocycle with trifluoromethanesulfonic anhydride in the presence of an organic base such as 2,6-lutidine and the like.
Ester derivative (9) can be obtained by dissolving the thus-obtained intermediate (8) in a solvent such as DMF and the like, mixing the solution with an alcohol such as ethanol and the like and a catalyst such as tetrakis(triphenylphosphine)palladium(0) and the like and subjecting the mixture to a heating reaction in the presence of, where necessary, an organic base such as diisopropylethylamine and the like under a carbon monoxide gas atmosphere.
The thus-obtained ester derivative (9) is dissolved in a solvent such as tetrahydrofuran (hereinafter THF) and the like, and reacting same with lithium aluminum hydride to give alcohol derivative (10).

In the formula (1), when R¹ is a group represented by the formula (2-1), ring A is an aliphatic nitrogen-containing heterocyclic group having 2 to 8 carbon atoms, W is a group represented by the formula (4), ring B is a nitrogen-containing heteroaryl group having 1 to 10 carbon atoms, or an aliphatic nitrogen-containing heterocyclic group having 2 to 8 carbon atoms, and Y¹ is a single bond, carboxylic acid derivative (12) as an intermediate can be obtained by the method shown below.

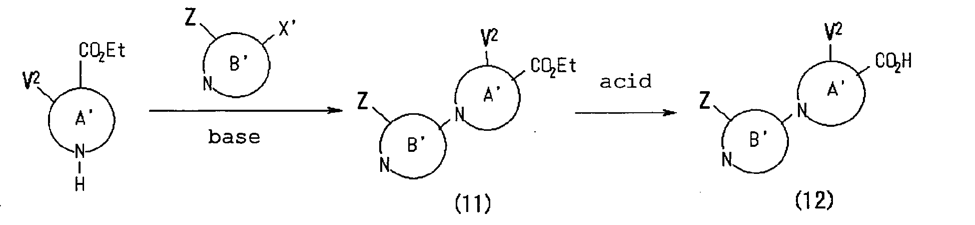

wherein X' is a leaving group such as a halogen atom and the like, ring A' is an aliphatic nitrogen-containing heterocyclic group having 2 to 8 carbon atoms, and ring B' is a nitrogen-containing heteroaryl group having 1 to 10 carbon atoms, or an aliphatic nitrogen-containing heterocyclic group having 2 to 8 carbon atoms, and other symbols are as defined above.
For example, intermediate (11) can be obtained by dissolving a compound having an aliphatic nitrogen-containing heterocycle such as ethyl isonipecotate and the like in a solvent such as xylene and the like, adding a nitrogen-containing heterocyclic compound having a leaving group such as a halogen atom and the like (e.g., chloropyridine and the like), and heating the mixture in the presence of an organic base such as triethylamine and the like.
The thus-obtained intermediate (11) is hydrolyzed under acidic conditions to give carboxylic acid derivative (12).

In the formula (1), when R¹ is a group represented by the formula (2-1), ring A is a C₆₋₁₀ aryl group or a C₁₋₁₀ heteroaryl group, W is a group represented by the formula (4), ring B is a nitrogen-containing heteroaryl group having 1 to 10 carbon atoms or an aliphatic nitrogen-containing heterocyclic group having 2 to 8 carbon atoms, Y¹ is an oxygen atom, and Z is a guanidino group optionally substituted by C₁₋₆ alkyl group(s) or a C₁₋₆ alkyl group optionally having an imino group at the 1-position, carboxylic acid derivative (14) as an intermediate can be obtained by the method shown below.

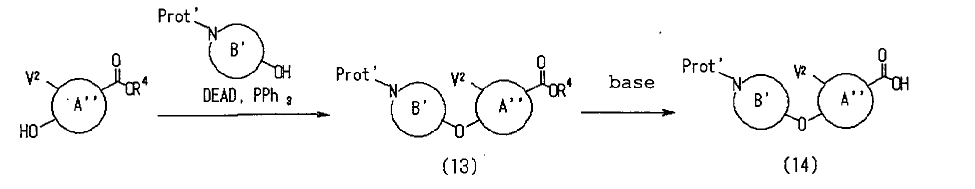

wherein Prot' is, an amino-protecting group (e.g., benzyloxycarbonyl group, a p-nitrobenzylcarbonyl group and the like), R⁴ is an alkyl group, ring A" is a C₆₋₁₀ aryl group or a C₁₋₁₀ heteroaryl group, and other symbols are as defined above.
For example, ether derivative (13) is obtained by dissolving arylcarboxylate or heteroarylcarboxylate having a hydroxyl group (e.g., ethyl 4-hydroxybenzoate and the like) and, for example, an aliphatic nitrogen-containing heterocycle having a hydroxyl group, wherein the nitrogen is protected by a suitable protecting group (Prot') such as a benzyloxycarbonyl group and the like that can be removed by catalytic reduction with palladium (e.g., N-tert-butoxycarbonyl-4-hydroxypiperidine and the like) in a solvent such as THF and the like, and reacting the heterocycle with diethylazodicarboxylic acid (DEAD) and triphenylphosphine.
The thus-obtained ether derivative (13) is hydrolyzed under basic conditions to give carboxylic acid derivative (14).

In the formula (1), when R¹ is a group represented by the formula (2-2), carboxylic acid derivative (15) as an intermediate can be obtained by the method shown below.

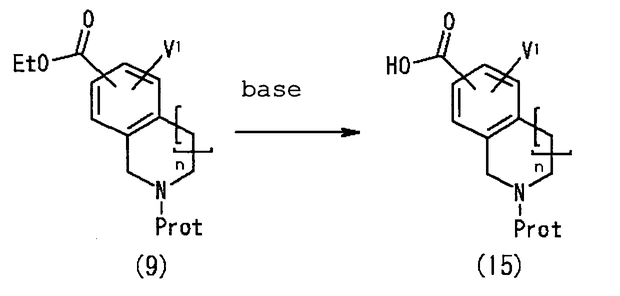

wherein each symbol is as mentioned above.
Carboxylic acid derivative (15) can be obtained by hydrolyzing ester derivative (9) under basic conditions.

In the formula (1), when R¹ is a group represented by the formula (2-2), intermediate (16) can be synthesized by the method shown below.

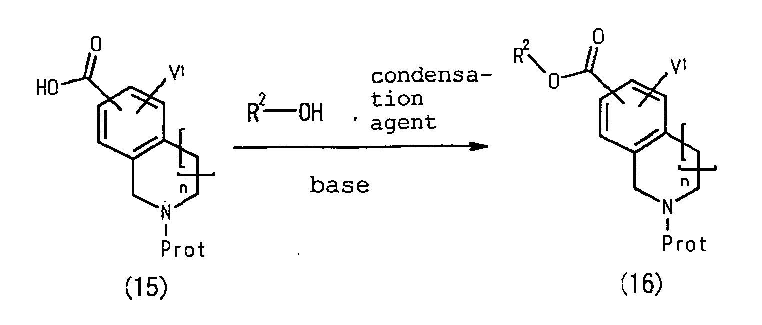

wherein each symbol is as defined above.
Intermediate (16) can be obtained by dissolving carboxylic acid derivative (15) and the corresponding alcohol derivative in a solvent such as dichloromethane, DMF and the like, and reacting the mixture with a condensation agent such as DCC, HATU and the like in the presence of, where necessary, an organic base such as triethylamine and the like.

In the formula (1), when R¹ is a group represented by the formula (2-2), ring A is an aliphatic nitrogen-containing heterocyclic group having 2 to 8 carbon atoms, W is a group represented by the formula (4), ring B is a nitrogen-containing heteroaryl group having 1 to 10 carbon atoms or an aliphatic nitrogen-containing heterocyclic group having 2 to 8 carbon atoms, and Y¹ is a single bond, alcohol derivative (17) as an intermediate can be synthesized by the method shown below.

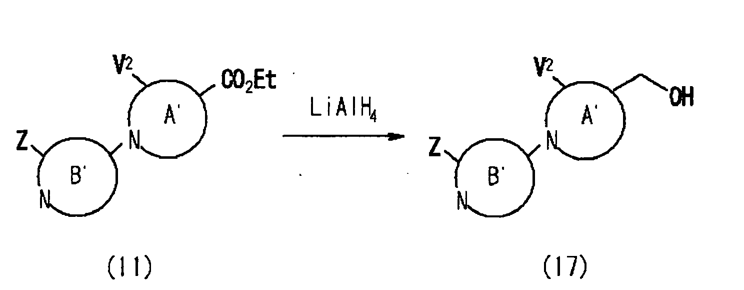

wherein each symbol is as defined above.
Alcohol derivative (17) can be obtained by dissolving ester derivative (11) in a solvent such as THF and the like, and reacting the derivative with lithium aluminum hydride.

In addition, in the formula (1), when R¹ is a group represented by the formula (2-2), ring A is a C₆₋₁₀ aryl group or a C₁₋₁₀ heteroaryl group, W is a group represented by the formula (4), ring B is a nitrogen-containing heteroaryl group having 1 to 10 carbon atoms, or an aliphatic nitrogen-containing heterocyclic group having 2 to 8 carbon atoms, and Y¹ is an oxygen atom, alcohol derivative (18) as an intermediate can be obtained by the method shown below.

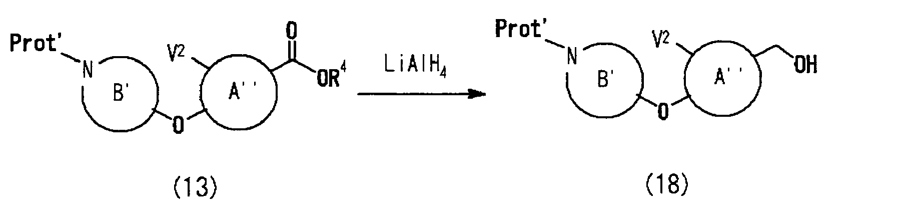

wherein each symbol is as defined above.
Alcohol derivative (18) can be obtained by dissolving ester derivative (13) in a solvent such as THF and the like, and reacting the derivative with lithium aluminum hydride.

Amidine derivative (20), which is compound (1) wherein X is a C₁₋₆ alkyl group, and guanidine derivative (21), which is compound (1) wherein X is an amino group can be synthesized by the following method.

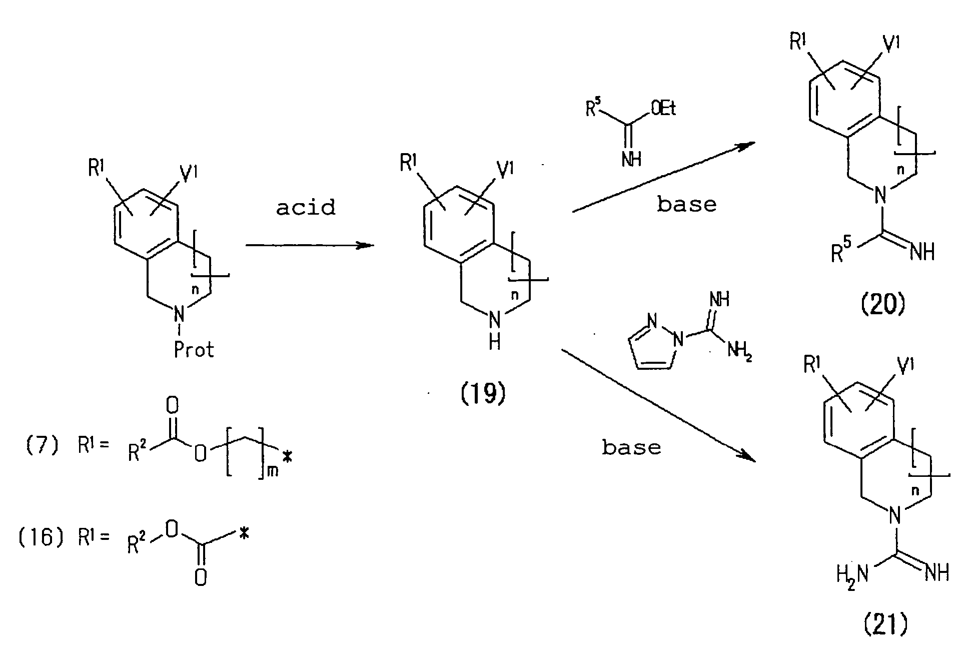

wherein R⁵ is a C₁₋₆ alkyl group, and other symbols are as defined above.
Intermediate (19) can be obtained by reacting intermediate (7) or (16) with an acid such as trifluoroacetic acid, a hydrochloric acid/1,4-dioxane solution and the like to remove the protecting group on the nitrogen.
Amidine derivative (20) can be obtained by dissolving the thus-obtained intermediate (19) in a solvent such as ethanol and the like, and reacting the intermediate with, for example, the corresponding imidate such as ethyl acetimidate and the like in the presence of, where necessary, an organic base such as diisopropylethylamine and the like.
In the same manner, guanidine derivative (21) can be obtained by reacting intermediate (19) with 1H-pyrazole-1-carboxamidine in the presence of, where necessary, an organic base such as diisopropylethylamine and the like.

Guanidine derivative (23), which is compound (1) wherein R¹ is a group represented by the formula (2-1), X is an amino group, ring A is a C₆₋₁₀ aryl group or a C₁₋₁₀ heteroaryl group, W is a group represented by the formula (4), ring B is a nitrogen-containing heteroaryl group having 1 to 10 carbon atoms, or an aliphatic nitrogen-containing heterocyclic group having 2 to 8 carbon atoms, Z is a C₁₋₆ alkyl group having an imino group at the 1-position, and Y¹ is an oxygen atom can be synthesized by the following method.

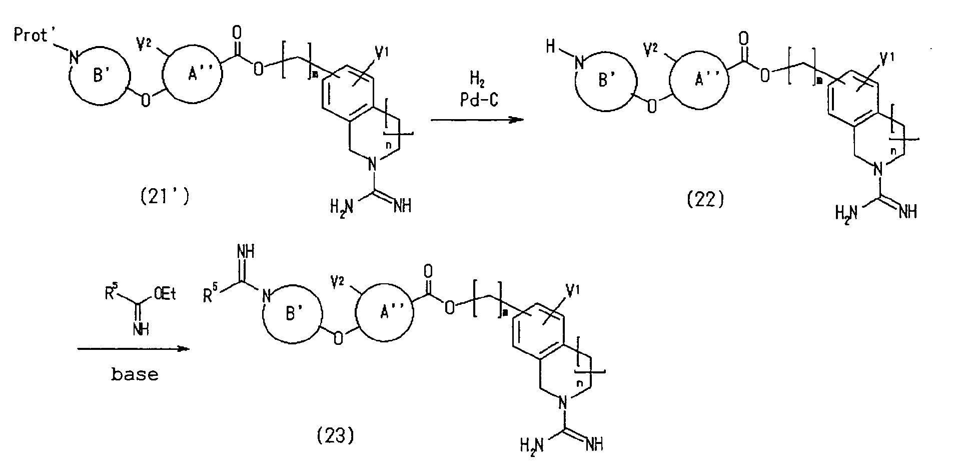

wherein each symbol is as defined above.
Intermediate (22) is obtained by dissolving intermediate (21') in a solvent such as alcohol (e.g., ethanol etc.), acetic acid and the like, and subjecting to a catalytic reaction under a hydrogen atmosphere with, for example, a palladium catalyst such as palladium/carbon and the like, to remove the protecting group of ring B.
The thus-obtained intermediate (22) is dissolved in a solvent such as ethanol, DMF and the like, and reacting the intermediate with, for example, the corresponding imidate such as ethyl acetimidate and the like in the presence of, where necessary, an organic base such as diisopropylethylamine and the like to give guanidine derivative (23).

In the same manner, guanidine derivative (25), which is compound (1) wherein R¹ is a group represented by the formula (2-2), X is an amino group, ring A is a C₆₋₁₀ aryl group or a C₁₋₁₀ heteroaryl group, W is a group represented by the formula (4), ring B is a nitrogen-containing heteroaryl group having 1 to 10 carbon atoms, or an aliphatic nitrogen-containing heterocyclic group having 2 to 8 carbon atoms, Z is a C₁₋₆ alkyl group having an imino group at the 1-position, and Y¹ is an oxygen atom can be obtained by the following method.

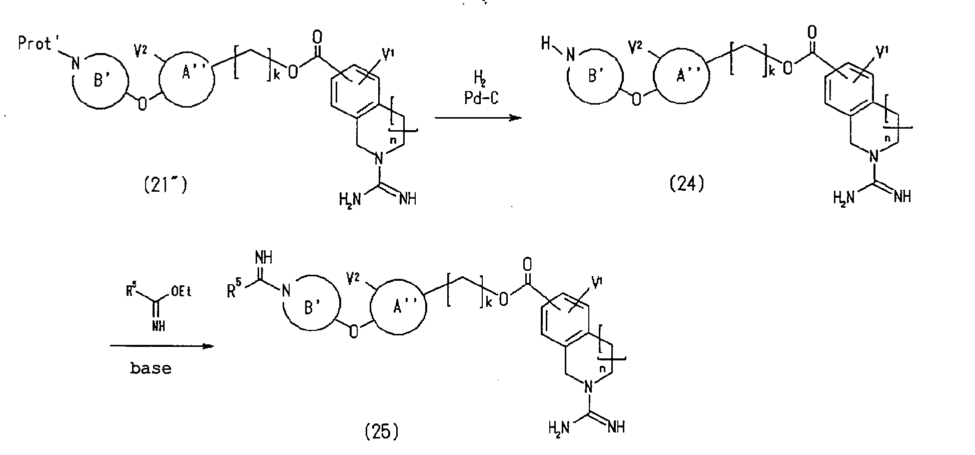

wherein each symbol is as defined above.

Compound (34), which is compound (5) wherein R¹ is a group represented by the formula (2-1), V³ is a group represented by the formula (6), R³ is a C₁₋₆ alkyl group optionally having substituent(s) or a C₃₋₁₀ cycloalkyl group optionally having substituent(s), Y² is -CONH-, Y³ is -(CH₂)ᵢ-, k is 1, j is 1, and i is 0 can be synthesized by the following method.

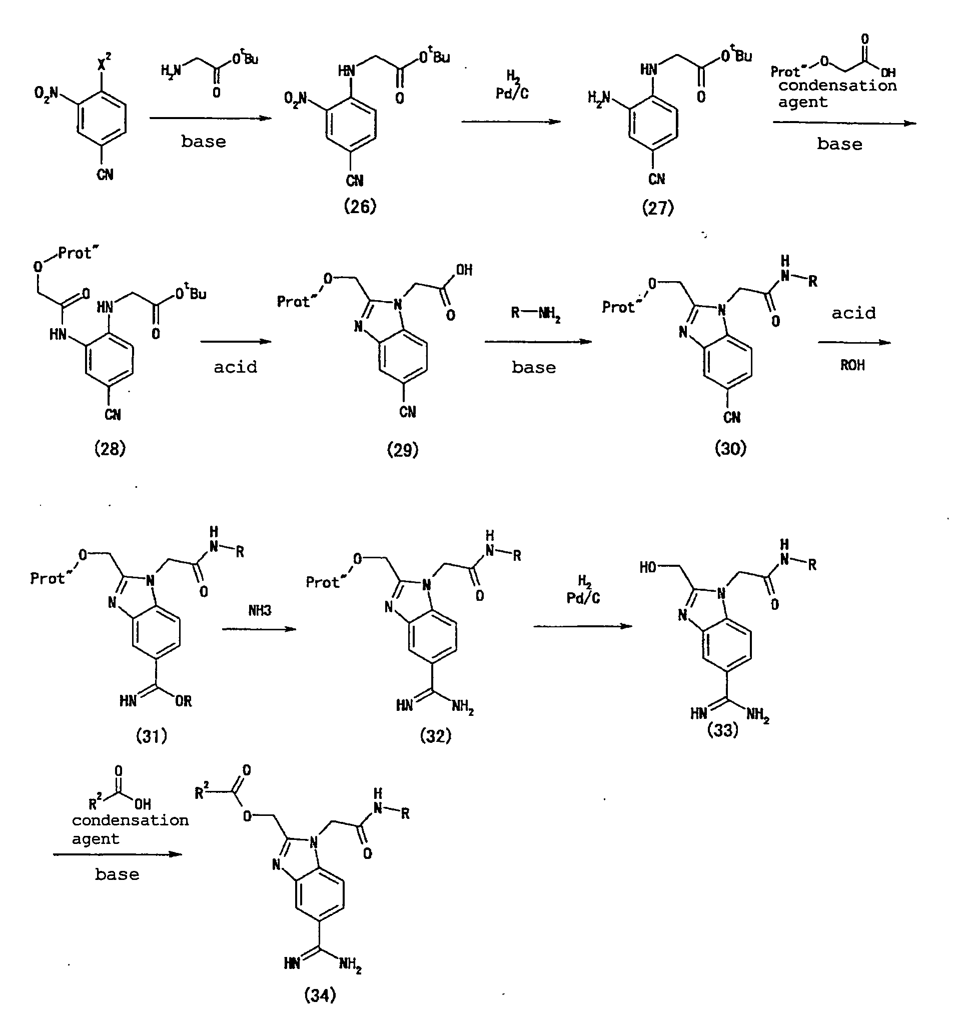

wherein Prot" is a hydroxyl-protecting group (e.g., a benzyl group, a p-methoxybenzyl group and the like), X² is a leaving group such as a halogen atom and the like, R are each independently a C₁₋₆ alkyl group optionally having substituent(s) or a C₃₋₁₀ cycloalkyl group optionally having substituent(s), and other symbols are as defined above.
For example, intermediate (26) can be obtained by reacting 3-nitrobenzonitrile having a leaving group such as a halogen atom and the like at the 4-position with glycine tert-butyl ester in alcohol such as ethanol and the like as a solvent and in the presence of an organic base such as triethylamine and the like.
The nitro group of intermediate (26) is reduced to an amino group by a catalytic reduction under a hydrogen atmosphere by, for example, a palladium catalyst such as palladium carbon and the like, and the like to give intermediate (27), which is dissolved in a solvent such as DMF and the like and reacted with, for example, a hydroxyacetic acid derivative wherein the hydroxyl group is protected with a protecting group (Prot") that can be removed by a catalytic reduction by a palladium catalyst under a hydrogen atmosphere, such as 2-benzyloxyacetic acid and the like, in the presence of an organic base such as triethylamine and the like and using, where necessary, a condensation agent such as HATU, HOAt and the like to give intermediate (28).
The thus-obtained (28) was heated under acidic conditions such as acetic acid, methanesulfonic acid, p-toluenesulfonic acid and the like, and treated, where necessary, with an acid such as trifluoroacetic acid and the like to give benzimidazole intermediate (29).
The thus-obtained intermediate (29) is dissolved in a solvent such as DMF and the like, and reacted with the corresponding alkylamine and a condensation agent such as HATU, HOAt and the like in the presence of an organic base such as triethylamine and the like to give intermediate (30).
The thus-obtained (30) is dissolved in, for example, alcohol such as methanol, ethanol and the like as a solvent, and reacted with an acid such as a hydrochloric acid/1,4-dioxane solution and the like or, for example, hydrogen chloride gas as an acid is blown in to give imidate (31).
The thus-obtained imidate (31) is treated with an ammonium salt such as ammonium carbonate and the like using, for example, alcohol such as ethanol and the like as a solvent or ammonia gas is blown in to give amidine intermediate (32).
The thus-obtained (32) is dissolved in alcohol such as ethanol and the like as a solvent and, under a hydrogen atmosphere, subjected to a catalytic reduction with, for example, a palladium catalyst such as palladium/carbon and the like, to remove a protecting group, whereby alcohol intermediate (33) can be obtained.
The thus-obtained (33) is dissolved in a solvent such as DMF, dichloromethane and the like, and reacted with the corresponding carboxylic acid and a condensation agent, or the corresponding carboxylic acid is led to acid chloride with phosphorus oxychloride and the like, and reacted with alcohol intermediate (33) to give compound (34).

When the compounds represented by the formulas (1) and (5) of the present invention can form a salt, the salt may be any as long as it is pharmaceutically acceptable. Examples thereof with an acidic group in the case an acidic group such as a carboxyl group and the like is present in the formula include ammonium salt, salts with alkali metals such as sodium, potassium and the like, salts with alkaline earth metals such as calcium, magnesium and the like, aluminum salt, zinc salt, salts with organic amines such as triethylamine, ethanolamine, morpholine, piperidine, dicyclohexylamine and the like, salts with basic amino acids such as arginine, lysine and the like.

Examples thereof with a basic group in the case a basic group is present in the formula include salts with inorganic acids such as hydrochloric acid, sulfuric acid, phosphoric acid, nitric acid, hydrobromic acid and the like, salts with organic carboxylic acids such as acetic acid, trifluoroacetic acid, citric acid, benzoic acid, maleic acid, fumaric acid, tartaric acid, succinic acid, tannic acid, butyric acid, hibenzoic acid, pamoic acid, enanthic acid, decane acid, teoclic acid, salicylic acid, lactic acid, oxalic acid, mandelic acid, malic acid and the like, salts with organic sulfonic acids such as methanesulfonic acid, benzenesulfonic acid, p-toluenesulfonic acid and the like. A method for formation of the salt includes mixing a compound of the formula (1) or (5) and a necessary acid or base at a suitable amount ratio in a solvent or dispersing agent, or cation exchange or anion exchange from other salt form.

The compound of the present invention also includes solvates, for example, hydrate, alcohol adduct and the like, of the compounds represented by the formulas (1) and (5).

The compound of the present invention may be converted to a prodrug. The prodrug of the present invention means a compound that is converted in the body to produce the compound of the present invention. For example, when an active form contains a carboxyl group or a phosphoric acid group, ester thereof, amide thereof and the like can be mentioned. In addition, when the active form contains an amino group, examples thereof include amide thereof, carbamate thereof and the like. When the active form contains a hydroxyl group, examples thereof include ester thereof, carbonate thereof, carbamate thereof and the like. When the compound of the present invention is converted to a prodrug, it may be bonded to amino acid or saccharide.

The compound of the present invention, compound (1) or (5), or a pharmaceutically acceptable salt thereof can be administered as it is, or produced as a pharmaceutical composition according to a conventional method and using general preparation additives and administered. Examples of the dosage form of such pharmaceutical composition include tablet, powder, injection, freeze-dry injection, pill, granule, capsule, suppository, liquid, sugar coating agent, debot, syrup, suspension, emulsion, troche, hypoglottis, adhesive preparation, oral disintegrant (tablet), inhalant, enteroclysis, ointment, patch, tape, eye drop and the like.

The compound or pharmaceutical composition of the present invention is administered into a circuit for extracorporeal blood circulation or to patients. Preferable examples of the administration method include direct administration into the circuit for extracorporeal blood circulation, intravenous administration, intramuscular administration and subcutaneous administration. In some cases, oral administration, intrarectal administration, intranasal administration and sublingual administration can be performed. For direct administration into the circuit for extracorporeal blood circulation, it is preferably administered at a part of the circulation circuit that leads the blood outside the body, which is as close as possible to the body. In hemodialysis and the like, a usually-formed injection inlet can be utilized.

The subject of administration is not particularly limited, and examples thereof include mammals (e.g., mouse, rat, hamster, rabbit, cat, dog, swine, bovine, sheep, horse, monkey, human etc.) and the like.

In addition, to provide the compound of the present invention or a pharmaceutically acceptable salt thereof or a pharmaceutical composition containing same as an anti(blood)coagulation drug(agent) for hemodialysis, an FXa inhibitor composition per se to be used for a dialyzer by dissolution or dispersion in a dialysis solution when in use, or a dialysis solution or dialysis concentrate comprising an FXa inhibitor can be provided. Examples of the dialysis concentrate include powder preparation for artificial kidney, which can be prepared, for example, by concentrating a dialysis solution comprising an FXa inhibitor by freeze-drying and the like. The dialysis concentrate can be prepared into a dialysis solution before use by an appropriate method, for example, dilution with purified water.

The compound or pharmaceutical composition of the present invention is administered at once or in a sustained manner in one to several portions as necessary, for one time operation of extracorporeal blood circulation. While the dose of the compound or pharmaceutical composition of the present invention is 0.01 mg - 10 g, preferably 1 mg - 1000 mg, in the amount of the compound to be the active ingredient for one time operation of extracorporeal blood circulation or dose per day, it can be appropriately increased or decreased according to the age, body weight, symptom and the like of the patient/subject. In addition, while an appropriate concentration of the active ingredient compound in a dialysis solution varies depending on the compound to be used, severity of the disease to be treated and the characteristic of the patient to be treated, it is generally such concentration as achieves the average concentration in plasma of the usable compound at an appropriate equilibrium of 0.0001 - 1000 µmol/L, preferably 0.005 - 20 µmol/L.

### [Examples]

The present invention is explained in detail in the following by referring to Examples, which are not to be construed as limitative.

### Example 1 2-[amino(imino)methyl]-1,2,3,4-tetrahydroisoquinolin-7-yl 1-(pyridin-4-yl)piperidine-4-carboxylate ditrifluoroacetate

### step 1 Synthesis of 1,2,3,4-tetrahydroisoquinolin-7-ol acetate

To isoquinolin-7-ol (10.0 g, 68.9 mmol) and 86% platinum oxide (700 mg) was added acetic acid (200 mL), and the mixture was stirred at room temperature for 40 hr under a hydrogen atmosphere. The insoluble material was filtered off, and the solvent was evaporated under reduced pressure. To the obtained residue were added acetone and diethyl ether to allow precipitation of a solid to give the title compound.
yield (12.7 g, 60.5 mmol, 88%)
¹H-NMR (DMSO-d₆, 300MHz)δ1.88 (s, 3H), 2.55 (t, 2H), 2.90 (t, 2H), 3.75 (s, 2H), 6.38 (d, 1H), 6.50 (dd, 1H), 6.82 (d, 1H).

### step 2 Synthesis of tert-butyl 7-hydroxy-3,4-dihydroisoquinoline-2(1H)-carboxylate

To a solution (150 mL) of 1,2,3,4-tetrahydroisoquinolin-7-ol acetate (12.7 g, 60.5 mmol) in dichloromethane was added triethylamine (25 mL, 182 mmol), a solution (150 mL) of di-tert-butyl dicarbonate (13.2 g, 60.5 mmol) in dichloromethane was added dropwise under ice-cooling, and the mixture was directly stirred for 1 hr. The solvent was evaporated under reduced pressure, the obtained residue was diluted with ethyl acetate, washed with 1N hydrochloric acid, saturated aqueous sodium hydrogen carbonate and saturated brine, dried over anhydrous magnesium sulfate and concentrated under reduced pressure. To the obtained residue were added ethyl acetate and diethyl ether to allow precipitation of a solid to give the title compound.
yield (11.1 g, 44.4 mmol, 74%)
MS (ESI, m/z) 250 [M+H]⁺
¹H-NMR (DMSO-d₆)δ1.40 (s, 9H), 2.61 (t, 2H), 3.48 (t, 2H), 4.36 (s, 2H), 6.50 (s, 1H), 6.55 (dd, 1H), 6.91 (d, 1H).

### step 3 Synthesis of tert-butyl 7-({[1-(pyridin-4-yl)piperidin-4-yl]carbonyl}oxy)-3,4-dihydroisoquinoline-2(1H)-carboxylate trifluoroacetate

A solution (6 mL) of 1-pyridin-4-ylpiperidine-4-carboxylic acid (291 mg, 1.20 mmol) and N,N'-dicyclohexylcarbodiimide (hereinafter DCC) (273 mg, 1.32 mmol) in pyridine was stirred at room temperature for 30 min, t-butyl 7-hydroxy-3,4-dihydroisoquinoline-2(1H)-carboxylate (300 mg, 1.20 mmol) was added, and the mixture was further stirred at room temperature overnight. The reaction mixture was concentrated under reduced pressure and the obtained residue was subjected to reversed-phase HPLC using octadodecyl group-chemically bonded type silica gel as a filler, and eluted with a mixed solution of water and acetonitrile, which contained trifluoroacetic acid at 0.1% (v/v). The object fraction was lyophilized to give the title compound.
yield (349 mg, 44.4 mmol, 53%)
MS (ESI, m/z) 438 [M+H]⁺
¹H-NMR (DMSO-d₆)δ1.42 (9H, s), 1.71-1.81 (2H, m), 2.11-2.15 (2H, m), 2.77 (2H, t), 3.04-3.12 (1H, m), 3.40 (2H, t), 3.55 (2H, t), 4.18-4.22 (2H, m), 4.49 (2H, s), 6.93-6.97 (2H, m), 7.19-7.25 (3H, m), 8.25 (2H, d).

### step 4 Synthesis of 2-[amino(imino)methyl]-1,2,3,4-tetrahydroisoquinolin-7-yl 1-(pyridin-4-yl)piperidine-4-carboxylate ditrifluoroacetate

To a solution (0.2 mL) of tert-butyl 7-({[1-(pyridin-4-yl)piperidin-4-yl]carbonyl}oxy)-3,4-dihydroisoquinoline-2(1H)-carboxylate trifluoroacetate (50 mg, 0.097 mmol) in 1,4-dioxane was added 4N hydrochloric acid/1,4-dioxane solution (0.8 mL), and the mixture was stirred at room temperature for 1 hr. The solvent was evaporated under reduced pressure, and the obtained residue was dissolved in dehydrated DMF (1 mL). 1H-Pyrazole-1-carboxamidine hydrochloride (16 mg, 0.109 mmol) and diisopropylethylamine (0.047 mL, 0.272 mmol) were added, and the mixture was stirred at room temperature overnight and concentrated under reduced pressure. The obtained residue was purified by reversed-phase HPLC in the same manner as in step 3 to give the title compound.
yield (31.4 mg, 0.0517 mmol, 57%)
MS (ESI, m/z) 380 [M+H]⁺
¹H-NMR (DMSO-d₆)δ1.72-1.84 (m, 2H), 2.13-2.19 (m, 2H), 2.89-2.94 (m, 2H), 3.06-3.15 (m, 1H), 3.40 (t, 2H), 3.61-3.65 (m, 2H), 4.22 (d, 2H), 4.59 (s, 2H), 6.95 (d, 1H), 7.02 (dd, 1H), 7.24-7.31 (m, 3H), 7.67 (s, 4H), 8.26 (d, 2H).

### Example 2 [1-(pyridin-4-yl)piperidin-4-yl]methyl 2-[amino(imino)methyl]-1,2,3,4-tetrahydroisoquinoline-7-carboxylate ditrifluoroacetate

### step 1 Synthesis of ethyl 1-(pyridin-4-yl)piperidine-4-carboxylate

To a suspension (480 mL) of 1-(pyridin-4-yl)piperidine-4-carboxylic acid (20.0 g, 97.0 mmol) in ethanol was added dropwise thionyl chloride (7.1 mL, 97.0 mmol), and the mixture was stirred at 50°C overnight. The solvent was evaporated under reduced pressure, and the obtained residue was diluted with dichloromethane, washed with 1N aqueous sodium hydroxide solution and saturated aqueous ammonium chloride solution, dried over anhydrous sodium sulfate, and concentrated under reduced pressure to give the title compound without purification.
yield (21.0 g, 89.7 mmol, 93%)
MS (ESI, m/z) 235 [M+H]⁺
¹H-NMR (CDCl₃)δ1.26 (t, 3H), 1.72-1.86 (m, 2H), 1.97-2.05 (m, 2H), 2.49-2.58 (m, 1H), 2.93-3.02 (m, 2H), 3.82 (dt, 2H), 4.16 (q, 2H), 6.66 (dd, 2H), 8.25 (dd, 2H).

### step 2 Synthesis of [1-(pyridin-4-yl)piperidin-4-yl]methanol

A solution (4 mL) of ethyl 1-(pyridin-4-yl)piperidine-4-carboxylate (1.00 g, 4.27 mmol) in dehydrated THF was added dropwise to a suspension of lithium aluminum hydride (113 mg, 2.99 mmol) in dehydrated THF (10 mL) under ice-cooling, and the mixture was stirred at room temperature for 3 hr. Lithium aluminum hydride (30 mg) was added, and the mixture was further stirred at room temperature for 1 hr. Under ice-cooling, water (0.15 mL), 15% aqueous sodium hydroxide solution (0.15 mL) and water (0.45 mL) were added in this order, and the mixture was stirred at room temperature overnight. The insoluble material was filtered off, and the filtrate was concentrated under reduced pressure to give the title compound without purification.
yield (356 mg, 1.85 mmol, 43%)
MS (ESI, m/z) 193 [M+H]⁺
¹H-NMR (DMSO-d₆)δ1.13 (qd, 2H), 1.55-1.75 (m, 3H), 2.79 (td, 2H), 3.26 (t, 2H), 3.88-3.95 (m, 2H), 4.48 (t, 1H), 6.77-6.79 (m, 2H), 8.09-8.12 (m, 2H).

### step 3 Synthesis of 2-tert-butyl 7-{[1-(pyridin-4-yl)piperidin-4-yl]methyl} 3,4-dihydroisoquinoline-2,7(1H)-dicarboxylate

To [1-(pyridin-4-yl)piperidin-4-yl]methanol (55.5 mg, 0.288 mmol), 2-(tert-butoxycarbonyl)-1,2,3,4-tetrahydroisoquinoline-7-carboxylic acid (80.0 mg, 0.288 mmol) and HATU (110 mg, 0.288 mmol) was added DMF (1.9 mL), and the mixture was stirred at room temperature for 3 days. The mixture was concentrated under reduced pressure, and the residue was diluted with dichloromethane, washed with saturated aqueous sodium hydrogen carbonate solution and saturated brine, and dried over anhydrous magnesium sulfate. The solvent was evaporated under reduced pressure to give the title compound without purification.
yield (142 mg, 0.314 mmol, quantitative)
MS (ESI, m/z) 452 [M+H]⁺

### step 4 Synthesis of [1-(pyridin-4-yl)piperidin-4-yl]methyl 2-[amino(imino)methyl]-1,2,3,4-tetrahydroisoquinoline-7-carboxylate ditrifluoroacetate

An operation similar to that of Example 1, step 4, was performed using 2-tert-butyl 7-{[1-(pyridin-4-yl)piperidin-4-yl]methyl} 3,4-dihydroisoquinoline-2,7(1H)-dicarboxylate (142 mg, 0.314 mmol) as a starting material to give the title compound.
yield (134 mg, 0.216 mmol, 75%)
MS (ESI, m/z) 394 [M+H]⁺
¹H-NMR (DMSO-d₆)δ1.33 (q, 2H), 1.91 (d, 2H), 2.20 (br s, 1H), 3.00 (t, 2H), 3.21 (t, 2H), 3.63 (t, 2H), 4.19 (d, 2H), 4.28 (d, 2H), 4.66 (s, 2H), 7.21 (d, 2H), 7.41 (d, 1H), 7.54 (s, 4H), 7.76 (s, 1H), 7.84 (d, 1H), 8.22 (d, 2H), 13.36 (brs, 1H).

### Example 3 {2-[amino(imino)methyl]-1,2,3,4-tetrahydroisoquinolin-7-yl}methyl 1-(pyridin-4-yl)piperidine-4-carboxylate ditrifluoroacetate

### step 1 Synthesis of tert-butyl 7-{[(trifluoromethyl)sulfonyl]oxy}-3,4-dihydroisoquinoline-2(1H)-carboxylate

To a solution (20 mL) of tert-butyl 7-hydroxy-3,4-dihydroisoquinoline-2(1H)-carboxylate (1.00 g, 4.01 mmol) in dichloromethane were added 2,6-lutidine (0.56 mL, 4.81 mmol) and trifluoromethanesulfonic anhydride (0.742 mL, 4.41 mmol) under ice-cooling, and the mixture was stirred at room temperature for 3.5 hr. 1N Hydrochloric acid was added to the reaction mixture, and the mixture was extracted with dichloromethane, washed with 1N aqueous sodium hydroxide solution and saturated brine, dried over anhydrous magnesium sulfate and concentrated under reduced pressure to give the title compound without purification.
yield (1.59 g, 4.17 mmol, quantitative)
MS (ESI, m/z) 367 [(M-Me)+H]⁺

### step 2 Synthesis of 2-tert-butyl 7-ethyl 3,4-dihydroisoquinoline-2,7(1H)-dicarboxylate

To a solution (20 mL) of tert-butyl 7-{[(trifluoromethyl)sulfonyl]oxy}-3,4-dihydroisoquinoline-2(1H)-carboxylate (1.50 g, 3.93 mmol) in DMF were added ethanol (4 mL), tetrakis(triphenylphosphine)palladium(0) (227 mg, 0.197 mmol) and diisopropylethylamine (1.37 mL, 7.86 mmol), and the mixture was stirred at 70°C overnight under a carbon monoxide atmosphere. The insoluble material was removed by filtration, and the solvent was evaporated under reduced pressure. The obtained residue was purified by silica gel chromatography (hexane:ethyl acetate 100:0 - 88:12) to give the title compound.
yield (1.10 g, 3.60 mmol, 90%)
MS (ESI, m/z) 291 [(M-Me)+H]⁺
¹H-NMR (CDCl₃)δ1.37 (t, 3H), 1.50 (s, 9H), 2.88 (t, 2H), 3.66 (t, 2H), 4.37 (q, 2H), 4.61 (s, 2H), 7.20 (d, 1H), 7.80-7.85 (m, 2H).

### step 3 Synthesis of tert-butyl 7-(hydroxymethyl)-3,4-dihydroisoquinoline-2(1H)-carboxylate

An operation similar to that of Example 2, step 2, was performed using 2-tert-butyl 7-ethyl 3,4-dihydroisoquinoline-2,7(1H)-dicarboxylate (300 mg, 0.982 mmol) as a starting material to give the title compound without purification.
yield (232 mg, 0.881 mmol, 90%)
MS (ESI, m/z) 249 [(M-Me)+H]⁺
¹H-NMR (CDCl₃)δ1.49 (s, 9H), 2.83 (t, 2H), 3.64 (t, 2H), 4.58 (s, 2H), 4.66 (d, 2H), 7.13-7.15 (m, 3H).

### step 4 Synthesis of tert-butyl 7-[({[1-(pyridin-4-yl)piperidin-4-yl]carbonyl}oxy)methyl]-3,4-dihydroisoquinoline-2(1H)-carboxylate

An operation similar to that of Example 2, step 3, was performed using 1-(pyridin-4-yl)piperidine-4-carboxylic acid (90.8 mg, 0.440 mmol) and tert-butyl 7-(hydroxymethyl)-3,4-dihydroisoquinoline-2(1H)-carboxylate (116 mg, 0.440 mmol) as starting materials to give the title compound without purification.
yield (234 mg, 0.518 mmol, quantitative)
MS (ESI, m/z) 452 [M+H]⁺

### step 5 Synthesis of {2-[amino(imino)methyl]-1,2,3,4-tetrahydroisoquinolin-7-yl}methyl 1-(pyridin-4-yl)piperidine-4-carboxylate ditrifluoroacetate

An operation similar to that of Example 1, step 4, was performed using tert-butyl 7-[({[1-(pyridin-4-yl)piperidin-4-yl]carbonyl}oxy)methyl]-3,4-dihydroisoquinoline-2(1H)-carboxylate (234 mg, 0.518 mmol) as a starting material to give the title compound.
yield (189 mg, 0.304 mmol, 69%)
MS (ESI, m/z) 394 [M+H]⁺
¹H-NMR (DMSO-d₆)δ1.54-1.68 (m, 2H), 1.96-2.03 (m, 2H), 2.80-2.93 (m, 3H), 3.25-3.35 (m, 2H), 3.60 (t, 2H), 4.10-4.18 (m, 2H), 4.57 (s, 2H), 5.10 (s, 2H), 7.15 (s, 1H), 7.20 (d, 2H), 7.25 (s, 2H), 7.50 (s, 4H), 8.22 (d, 2H).

### Example 4 2-[amino(imino)methyl]-1,2,3,4-tetrahydroisoquinolin-7-yl 4-(pyrrolidin-1-ylcarbonyl)benzoate trifluoroacetate

### step 1 Synthesis of tert-butyl 7-{[4-(pyrrolidin-1-ylcarbonyl)benzoyl]oxy}-3,4-dihydroisoquinoline-2(1H)-carboxylate

To 4-(pyrrolidin-1-ylcarbonyl)benzoic acid (263 mg, 1.20 mmol) and DCC (273 mg, 1.32 mmol) was added dehydrated pyridine (4 mL), and the mixture was stirred at room temperature for 30 min. tert-Butyl 7-hydroxy-3,4-dihydroisoquinoline-2(1H)-carboxylate (300 mg, 1.20 mmol) was added, and the mixture was stirred at room temperature overnight. HOAt (81.7 mg, 0.60 mmol) was added, the mixture was further stirred for two nights, and the solvent was evaporated under reduced pressure. The obtained residue was diluted with ethyl acetate, washed with 1N hydrochloric acid, saturated aqueous sodium hydrogen carbonate solution and saturated brine, dried over anhydrous magnesium sulfate and concentrated under reduced pressure. The residue was purified by silica gel chromatography (hexane:ethyl acetate 98:2 - 20:80) to give the title compound.
yield (156 mg, 0.346 mmol, 29%)
MS (ESI, m/z) 451 [M+H]⁺
¹H-NMR (CDCl₃)δ1.49 (s, 9H), 1.89-2.02 (m, 2H), 2.83-2.87 (m, 2H), 3.39-3.43 (m, 2H), 3.66-3.70 (m, 4H), 4.60 (s, 2H), 6.98-7.04 (m, 2H), 7.18-7.21 (m, 1H), 7.62-7.66 (m, 2H), 8.21-8.25 (m, 2H).

### step 2 Synthesis of 2-[amino(imino)methyl]-1,2,3,4-tetrahydroisoquinolin-7-yl 4-(pyrrolidin-1-ylcarbonyl)benzoate trifluoroacetate

An operation similar to that of Example 1, step 4, was performed using tert-butyl 7-{[4-(pyrrolidin-1-ylcarbonyl)benzoyl]oxy}-3,4-dihydroisoquinoline-2(1H)-carboxylate (154 mg, 0.342 mmol) as a starting material to give the title compound.
yield (64.5 mg, 0.127 mmol, 37%)
MS (ESI, m/z) 393 [M+H]⁺
¹H-NMR (DMSO-d₆)δ1.82-1.92 (m, 4H), 2.94-2.97 (m, 2H), 3.36 (t, 2H), 3.50 (t, 2H), 3.64 (t, 2H), 4.61 (s, 2H), 7.12 (s, 1H), 7.20 (dd, 1H), 7.36 (d, 1H), 7.49 (s, 4H), 7.72 (d, 2H), 8.17 (d, 2H).

### Example 5 2-[amino(imino)methyl]-1,2,3,4-tetrahydroisoquinolin-7-yl 4-[(benzyloxy)methyl]-1-(pyridin-4-yl)piperidine-4-carboxylate ditrifluoroacetate

### step 1 Synthesis of 4-[(benzyloxy)methyl]-1-(pyridin-4-yl)piperidine-4-carboxylic acid

Ethyl 1-(pyridin-4-yl)piperidine-4-carboxylate (1.0 g, 4.26 mmol) was dissolved in THF (10 mL), and 1.8 M lithiumdiisopropylamide (2.6 mL) was added dropwise at -78°C. After stirring for 30 min, benzyloxymethyl chloride (0.6 mL, 4.26 mmol) was added dropwise. After stirring at room temperature for 2 hr, the mixture was worked up by a conventional method to give an intermediate. The obtained intermediate (200 mg, about 0.56 mmol) was dissolved in 6 N aqueous sodium hydroxide solution (2 mL), THF (2 mL) and ethanol (1 mL), and the mixture was stirred at 50°C overnight. After evaporation of the solvent, water (3 mL) was added, and the mixture was neutralized with concentrated hydrochloric acid. The precipitated solid was collected by filtration or the mixture was extracted with ethyl acetate to give the title compound.
yield (120 mg, 66%)
MS (ESI, m/z) 327[M+H]⁺
¹H-NMR(DMSO-d₆)δ8.10 (d, 2H), 7.30-7.35 (m, 5H), 6.80 (d, 2H), 4.45 (s, 2H), 3.50-3.80 (m, 2H), 3.00-3.45 (m, 4H), 1.89-2.02 (m, 2H), 1.45-1.56 (m, 2H).

### step 2 Synthesis of tert-butyl 7-[({4-[(benzyloxy)methyl]-1-(pyridin-4-yl)piperidin-4-yl}carbonyl)oxy]-3,4-dihydroisoquinoline-2(1H)-carboxylate

4-[(Benzyloxy)methyl]-1-(pyridin-4-yl)piperidine-4-carboxylic acid (57 mg, 0.18 mmol), tert-butyl 7-hydroxy-3,4-dihydroisoquinoline-2(1H)-carboxylate (75 mg, 0.18 mmol), DCC (57 mg, 0.27 mmol) and DMAP (42 mg) were mixed in dichloromethane (2 mL), and the mixture was stirred at room temperature overnight. The insoluble material was removed by filtration, and the solvent was evaporated. The obtained residue was purified by reversed-phase HPLC in the same manner as in Example 1, step 3, to give the title compound.
yield (107 mg, quantitative) MS (ESI, m/z) 458[M+H]⁺

### step 3 Synthesis of 2-[amino(imino)methyl]-1,2,3,4-tetrahydroisoquinolin-7-yl 4-[(benzyloxy)methyl]-1-(pyridin-4-yl)piperidine-4-carboxylate ditrifluoroacetate

An operation similar to that of Example 1, step 4, was performed using tert-butyl 7-[({4-[(benzyloxy)methyl]-1-(pyridin-4-yl)piperidin-4-yl}carbonyl)oxy]-3,4-dihydroisoquinoline-2(1H)-carboxylate (107mg) to give the title compound.
yield (68.5 mg, 77%)
MS (ESI, m/z) 500[M+H]⁺
¹H-NMR(D₂O)δ8.05 (d, 2H), 7.43 (m, 5H) 7.35 (d, 1H), 7.05 (d, 2H), 6.97-6.94 (m, 1H), 6.87 (m, 1H), 4.66 (s, 2H), 4.55 (s, 2H), 4.04-3.96 (m, 2H), 3.85 (m, 2H), 3.66 (m, 2H), 3.65-3.47 (m, 2H), 3.04-2.99 (m, 2H), 2.46-2.41 (m, 2H), 1.91-1.78 (m, 2H).

### Example 6 2-[amino(imino)methyl]-1,2,3,4-tetrahydroisoquinolin-7-yl 4-methoxymethyl-1-(pyridin-4-yl)piperidine-4-carboxylate ditrifluoroacetate

An operation similar to that of Example 5, step 1, was performed using methoxymethyl chloride instead of benzyloxymethyl chloride to give an intermediate. Furthermore, an operation similar to that of Example 5, step 2-step 3, was performed to give the title compound.
MS (ESI, m/z) 424[M+H]⁺
¹H-NMR(D₂O, 300MHz)δ8.06 (d, 2H), 7.35 (d, 1H), 7.04 (d, 2H), 6.97-6.94 (m, 1H), 6.87 (m, 1H), 4.54 (s, 2H), 4.04-3.96 (m, 2H), 3.85 (m, 2H), 3.66 (m, 2H), 3.65-3.47 (m, 5H), 3.04-2.99 (m, 2H), 2.49-2.40 (m, 2H), 1.92-1.75 (m, 2H).

### Example 7 [1-(pyridin-4-yl)piperidin-4-yl]methyl 2-[amino(imino)methyl]isoindoline-5-carboxylate ditrifluoroacetate

### step 1 Synthesis of tert-butyl 5-bromo-1,3-dihydro-2H-isoindole-2-carboxylate

To a solution (45 mL) of 5-bromoisoindoline (1.78 g, 9.00 mmol) in dichloromethane were added triethylamine (1.25 mL, 9.00 mmol) and di-tert-butyl dicarbonate (1.96 g, 9.00 mmol) under ice-cooling, and the mixture was stirred at room temperature for 2.5 hr. N,N-Dimethylethylamine (0.988 mL, 9.00 mmol) was added under ice-cooling, the mixture was stirred at room temperature for 1 hr, and 1 N hydrochloric acid was added to the reaction mixture. The mixture was extracted with dichloromethane, and the extract was washed with 1 N hydrochloric acid, saturated aqueous sodium hydrogen carbonate solution and saturated brine, dried over anhydrous magnesium sulfate, and concentrated under reduced pressure. The obtained residue was purified by silica gel chromatography (hexane:ethyl acetate 100:0 - 92:8) to give the title compound.
yield (1.72 g, 5.76 mmol, 64%)
MS (ESI, m/z) 284 [(M-Me)+H]⁺
¹H-NMR (CDCl₃)δ1.51 (s, 9H), 4.59-4.67 (m, 4H), 7.08-7.15 (m, 1H), 7.36-7.42 (m, 2H).

### step 2 Synthesis of 2-tert-butyl 5-ethyl 1,3-dihydro-2H-isoindole-2,5-dicarboxylate

An operation similar to that of Example 3, step 2, was performed using tert-butyl 5-bromo-1,3-dihydro-2H-isoindole-2-carboxylate (1.71 g, 5.73 mmol) as a starting material to give the title compound.
yield (609 mg, 2.09 mmol, 37%)
¹H-NMR (CDCl₃)δ1.39-1.45 (m, 3H), 1.53-1.54 (m, 9H), 4.36-4.43 (m, 2H), 4.70-4.74 (m, 4H), 7.26-7.36 (m, 1H), 7.93-8.00 (m, 2H).

### step 3 Synthesis of 2-(tert-butoxycarbonyl)isoindoline-5-carboxylic acid

To 2-tert-butyl 5-ethyl 1,3-dihydro-2H-isoindole-2,5-dicarboxylate (300 mg, 1.03 mmol) were added THF (1 mL) and ethanol (1 mL), and 2 M aqueous sodium hydroxide solution was added under ice-cooling. After stirring at room temperature for 1.5 hr, 2 N hydrochloric acid (1 mL) was added, and the mixture was concentrated under reduced pressure. The residue was diluted with ethyl acetate, washed with 0.1 N hydrochloric acid, and dried over anhydrous sodium sulfate. The solvent was evaporated under reduced pressure to give the title compound without purification.
yield (283 mg, 1.07 mmol, quantitative)

### step 4 Synthesis of 2-tert-butyl 5-1[1-(pyridin-4-yl)piperidin-4-yl]methyl} 1,3-dihydro-2H-isoindole-2,5-dicarboxylate

An operation similar to that of Example 2, step 3, was performed using 2-(tert-butoxycarbonyl)isoindoline-5-carboxylic acid (138 mg, 0.526 mmol) and [1-(pyridin-4-yl)piperidin-4-yl]methanol (101 mg, 0.526 mmol) as starting materials to give the title compound without purification.
yield (376 mg, quantitative)
MS (ESI, m/z) 438 [M+H]⁺

### step 5 Synthesis of [1-(pyridin-4-yl)piperidin-4-yl]methyl 2-[amino(imino)methyl]isoindoline-5-carboxylate ditrifluoroacetate

An operation similar to that of Example 1, step 4, was performed using 2-tert-butyl 5-{[1-(pyridin-4-yl)piperidin-4-yl]methyl} 1,3-dihydro-2H-isoindole-2,5-dicarboxylate (376 mg, 0.526 mmol) as a starting material to give the title compound.
yield (175 mg, 0.288 mmol, 55%)
MS (ESI, m/z) 380 [M+H]⁺
¹H-NMR (DMSO-d₆)δ1.26-1.41 (m, 2H), 1.87-1.96 (m, 2H), 2.15-2.28 (m, 1H), 3.21 (t, 2H), 4.21 (d, 2H), 4.28 (d, 2H), 4.82 (s, 4H), 7.21 (d, 2H), 7.50-7.56 (m, 5H), 7.93-7.98 (m, 2H), 8.22 (d, 2H).

### Example 8 {2-[amino(imino)methyl]-isoindolin-5-yl}methyl 1-(pyridin-4-yl)piperidine-4-carboxylate ditrifluoroacetate

### step 1 Synthesis of tert-butyl 5-(hydroxymethyl)-1,3-dihydro-2H-isoindole-2-carboxylate

An operation similar to that of Example 2, step 2, was performed using 2-tert-butyl 5-ethyl 1,3-dihydro-2H-isoindole-2,5-dicarboxylate (300 mg, 1.03 mmol) as a starting material to give the title compound without purification.
yield (247 mg, 0.991 mmol, 96%)
MS (ESI, m/z) 250 [M+H]⁺

### step 2 Synthesis of tert-butyl 5-[({[1-(pyridin-4-yl)piperidin-4-yl]carbonyl}oxy)methyl]-1,3-dihydro-2H-isoindole-2-carboxylate

An operation similar to that of Example 2, step 3, was performed using 1-(pyridin-4-yl)piperidine-4-carboxylic acid (82.7 mg, 0.401 mmol) and tert-butyl 5-(hydroxymethyl)-1,3-dihydro-2H-isoindole-2-carboxylate (100 mg, 0.401 mmol) as starting materials to give the title compound without purification.
yield (214 mg, quantitative)
MS (ESI, m/z) 438 [M+H]⁺

### step 3 Synthesis of {2-[amino(imino)methyl]-isoindolin-5-yl}methyl 1-(pyridin-4-yl)piperidine-4-carboxylate ditrifluoroacetate

An operation similar to that of Example 1, step 4, was performed using tert-butyl 5-[({[1-(pyridin-4-yl)piperidin-4-yl]carbonyl}oxy)methyl]-1,3-dihydro-2H-isoindole-2-carboxylate (214 mg, 0.401 mmol) as a starting material to give the title compound.
yield (143 mg, 0.288 mmol, 59%) (2 steps)
MS (ESI, m/z) 380 [M+H]⁺
¹H-NMR (DMSO-d₆)δ1.54-1.68 (m, 2H), 1.96-2.03 (m, 2H), 2.82-2.92 (m, 1H), 3.25-3.35 (m, 2H), 4.11-4.19 (m, 2H), 4.74 (s, 4H), 5.15 (s, 2H), 7.20 (d, 2H), 7.34-7.40 (m, 3H), 7.49 (s, 4H), 8.23 (d, 2H).

### Example 9 2-[amino(imino)methyl]-2,3,4,5-tetrahydro-1H-2-benzazepin-8-yl 1-(pyridin-4-yl)piperidine-4-carboxylate ditrifluoroacetate

### step 1 Synthesis of 2,3,4,5-tetrahydro-1H-2-benzazepin-8-ol hydrobromide

To a solution (6 mL) of 8-methoxy-2,3,4,5-tetrahydro-1H-2-benzazepin-1-on (1.01 g, 5.28 mmol) in 1,4-dioxane was added dropwise a suspension (12 mL) of lithium aluminum hydride (701 mg, 18.5 mmol) in THF under ice-cooling, and the mixture was heated under reflux for 3.5 hr. The reaction mixture was ice-cooled, water (0.7 mL), 15% aqueous sodium hydroxide solution (0.7 mL) and water (2.1 mL) were added in this order, and the mixture was stirred at room temperature overnight. The insoluble material was filtered off, and the filtrate was concentrated under reduced pressure. To the obtained residue was added concentrated hydrobromic acid (10 mL), and the mixture was heated under reflux for 5 hr. The mixture was concentrated under reduced pressure, ethanol and diethyl ether were added to the residue, and the precipitate was collected by filtration to give the title compound.
yield (1.06 g, 4.33 mmol, 82%)
MS (ESI, m/z) 164 [M+H]⁺

### step 2 Synthesis of tert-butyl 8-hydroxy-1,3,4,5-tetrahydro-2H-2-benzazepine-2-carboxylate

To a solution (10 mL) of 2,3,4,5-tetrahydro-1H-2-benzazepin-8-ol hydrobromide (1.06 g, 4.33 mmol) in dichloromethane were added a solution (10 mL) of triethylamine (1.81 mL, 13.0 mmol) and di-tert-butyl dicarbonate (945 mg, 4.33 mmol) in dichloromethane under ice-cooling, and the mixture was stirred at room temperature for 1.5 hr. Water was added to the reaction mixture, the mixture was extracted with dichloromethane, and the extract was washed with 0.1N hydrochloric acid, saturated aqueous sodium hydrogen carbonate solution and saturated brine, and dried over anhydrous magnesium sulfate. The solvent was evaporated under reduced pressure. The obtained residue was washed with hexane and ethyl acetate, and the precipitate was collected by filtration to give the title compound.
yield (881 mg, 3.35 mmol, 77%)
MS (ESI, m/z) 249 [(M-Me)+H]⁺
¹H-NMR (CDCl₃)δ1.40 (s, 9H), 1.68-1.74 (m, 2H), 2.84-2.88 (m, 2H), 3.66 (br s, 2H), 4.30-4.36 (m, 2H), 5.11 (br s, 1H), 6.61-6.65 (m, 1H), 6.70-6.85 (m, 1H), 6.99 (d, 1H).

### step 3 Synthesis of tert-butyl 8-({[1-(pyridin-4-yl)piperidin-4-yl]carbonyl}oxy)-1,3,4,5-tetrahydro-2H-2-benzazepine-2-carboxylate

An operation similar to that of Example 2, step 3, was performed using 1-(pyridin-4-yl)piperidine-4-carboxylic acid (47 mg, 0.228 mmol) and tert-butyl 8-hydroxy-1,3,4,5-tetrahydro-2H-2-benzazepine-2-carboxylate (60 mg, 0.228 mmol) as starting materials to give the title compound without purification.
yield (119 mg, quantitative)
MS (ESI, m/z) 452 [M+H]⁺

### step 4 Synthesis of 2-[amino(imino)methyl]-2,3,4,5-tetrahydro-1H-2-benzazepin-8-yl 1-(pyridin-4-yl)piperidine-4-carboxylate ditrifluoroacetate

An operation similar to that of Example 1, step 4, was performed using tert-butyl 8-({[(1-(pyridin-4-yl)piperidin-4-yl]carbonyl}oxy)-1,3,4,5-tetrahydro-2H-2-benzazepine-2-carboxylate (119 mg, 0.228 mmol) as a starting material to give the title compound.
yield (28.2 mg, 0.045 mmol, 20%) (2 steps)
MS (ESI, m/z) 394 [M+H]⁺
¹H-NMR (DMSO-d₆)δ1.68-1.81 (m, 4H), 2.09-2.18 (m, 2H), 2.96-3.02 (m, 2H), 3.07-3.15 (m, 1H), 3.35-3.43 (m, 2H), 3.71-3.77 (m, 2H), 4.16-4.26 (m, 2H), 4.63 (s, 2H), 7.00 (dd, 1H), 7.19-7.30 (m, 4H), 7.39 (s, 4H), 8.25 (d, 2H).

### Example 10 {2-[amino(imino)methyl]-2,3,4,5-tetrahydro-1H-2-benzazepin-8-yl}methyl 1-(pyridin-4-yl)piperidine-4-carboxylate ditrifluoroacetate

### step 1 Synthesis of ethyl 2-(tert-butoxycarbonyl)-2,3,4,5-tetrahydro-1H-2-benzazepine-8-carboxylate

An operation similar to that of Example 3, step 1 and step 2 was performed using tert-butyl 8-hydroxy-1,3,4,5-tetrahydro-2H-2-benzazepine-2-carboxylate (700 mg, 2.66 mmol) as a starting material to give the title compound.
yield (766 mg, 2.40 mmol, 90%)
MS (ESI, m/z) 320 [M+H]⁺
¹H-NMR (CDCl₃)5 1.36-1.41 (m, 12H), 1.78 (quint, 2H), 2.95-3.02 (m, 2H), 3.71 (br s, 2H), 4.33-4.48 (m, 4H), 7.21 (d, 1H), 7.83-7.96 (m, 2H).

### step 2 Synthesis of tert-butyl 8-(hydroxymethyl)-1,3,4,5-tetrahydro-2H-2-benzazepine-2-carboxylate

An operation similar to that of Example 2, step 2, was performed using ethyl 2-(tert-butoxycarbonyl)-2,3,4,5-tetrahydro-1H-2-benzazepine-8-carboxylate (407 mg, 1.27 mmol) as a starting material to give the title compound without purification.
yield (319 mg, 1.15 mmol, 91%)
¹H-NMR (CDCl₃)δ1.38 (s, 9H), 1.70-1.79 (m, 2H), 2.91-2.94 (m, 2H), 3.67 (br s, 2H), 4.36-4.41 (m, 2H), 4.64 (s, 2H), 7.10-7.26 (m, 3H).

### step 3 Synthesis of tert-butyl 8-[({[l-(pyridin-4-yl)piperidin-4-yl]carbonyl}oxy)methyl]-1,3,4,5-tetrahydro-2H-2-benzazepine-2-carboxylate

An operation similar to that of Example 2, step 3, was performed using 1-(pyridin-4-yl)piperidine-4-carboxylic acid (60 mg, 0.291 mmol) and tert-butyl 8-(hydroxymethyl)-1,3,4,5-tetrahydro-2H-2-benzazepine-2-carboxylate (80.7 mg, 0.291 mmol) as starting materials to give the title compound without purification.
yield (165 mg, 0.518 mmol, quantitative)
MS (ESI, m/z) 466 [M+H]⁺

### step 4 Synthesis of {2-[amino(imino)methyl]-2,3,4,5-tetrahydro-1H-2-benzazepin-8-yl}methyl 1-(pyridin-4-yl)piperidine-4-carboxylate ditrifluoroacetate

An operation similar to that of Example 1, step 4, was performed using tert-butyl 8-[({[1-(pyridin-4-yl)piperidin-4-yl]carbonyl}oxy)methyl]-1,3,4,5-tetrahydro-2H-2-benzazepine-2-carboxylate (165 mg, 0.291 mmol) as a starting material to give the title compound.
yield (97 mg, 0.153 mmol, 52%)
MS (ESI, m/z) 408 [M+H]⁺
¹H-NMR (DMSO-d₆)δ1.55-1.76 (m, 4H), 1.95-2.05 (m, 2H), 2.80-2.90 (m, 1H), 2.96-3.01 (m, 2H), 3.25-3.35 (m, 2H), 3.70-3.77 (m, 2H), 4.10-4.19 (m, 2H), 4.64 (s, 2H), 5.08 (s, 2H), 7,18-7.24 (m, 4H), 7.41 (s, 4H), 7.44 (s, 1H), 8.22 (d, 2H).

### Example 11 [1-(pyridin-4-yl)piperidin-4-yl]methyl 2-[amino(imino)methyl]-2,3,4,5-tetrahydro-1H-2-benzazepine-8-carboxylate ditrifluoroacetate

### step 1 Synthesis of 2-(tert-butoxycarbonyl)-2,3,4,5-tetrahydro-1H-2-benzazepine-8-carboxylic acid

An operation similar to that of Example 7, step 3, was performed using ethyl 2-(tert-butoxycarbonyl)-2,3,4,5-tetrahydro-1H-2-benzazepine-8-carboxylate (343 mg, 1.07 mmol) to give the title compound without purification.
yield (266 mg, 0.913 mmol, 85%)
MS (ESI, m/z) 292 [M+H]⁺
¹H-NMR (CDCl₃)δ1.40 (s, 9H), 1.80-1.82 (m, 2H), 2.98-3.06 (m, 2H), 3.73 (br s, 2H), 4.42-4.50 (m, 2H), 7.24-7.26 (m, 1H), 7.91-8.06 (m, 2H).

### step 2 Synthesis of 2-tert-butyl 8-{[1-(pyridin-4-yl)piperidin-4-yl]methyl} 1,3,4,5-tetrahydro-2H-2-benzazepine-2,8-dicarboxylate

An operation similar to that of Example 2, step 3, was performed using 2-(tert-butoxycarbonyl)-2,3,4,5-tetrahydro-1H-2-benzazepine-8-carboxylic acid (86.2 mg, 0.296 mmol) and [1-(pyridin-4-yl)piperidin-4-yl]methanol (56.9 mg, 0.296 mmol) as starting materials to give the title compound without purification.
yield (156 mg, quantitative)
MS (ESI, m/z) 466 [M+H]⁺

### step 3 Synthesis of [1-(pyridin-4-yl)piperidin-4-yl]methyl 2-[amino(imino)methyl]-2,3,4,5-tetrahydro-1H-2-benzazepine-8-carboxylate ditrifluoroacetate

An operation similar to that of Example 1, step 4, was performed using 2-tert-butyl 8-{[1-(pyridin-4-yl)piperidin-4-yl]methyl} 1,3,4,5-tetrahydro-2H-2-benzazepine-2,8-dicarboxylate (156 mg, 0.296 mmol) as a starting material to give the title compound.
yield (73 mg, 0.115 mmol, 39%)
MS (ESI, m/z) 408 [M+H]⁺
¹H-NMR (DMSO-d₆)δ1.26-11.40 (m, 2H), 1.61-1.68 (m, 2H), 1.87-1.96 (m, 2H), 2.12-2.26 (m, 1H), 3.04-3.10 (m, 2H), 3.21 (t, 2H), 3.72-3.79 (m, 2H), 4.18 (d, 2H), 4.27 (d, 2H), 4.73 (s, 2H), 7.21 (d, 2H), 7.40 (d, 2H), 7.44 (s, 4H), 7.82 (dd, 1H), 8.13 (d, 1H), 8.22 (d, 2H).

### Example 12 2-[amino(imino)methyl]-1,2,3,4-tetrahydroisoquinolin-7-yl 4-({1-[amino(imino)methyl]piperidin-4-yl}oxy)benzoate ditrifluoroacetate

step 1 Synthesis of tert-butyl 7-[(4-{[1-(tert-butoxycarbonyl)piperidin-4-yl]oxy}benzoyl)oxy]-3,4-dihydroisoquinoline-2(1H)-carboxylate
4-{[1-(tert-Butoxycarbonyl)piperidin-4-yl]oxy}benzoic acid (200 mg, 0.622 mmol), tert-butyl 7-hydroxy-3,4-dihydroisoquinoline-2(1H)-carboxylate (155 mg, 0.622 mmol), DCC (141 mg, 0.684 mmol) and DMAP (114 mg, 0.933 mmol) were mixed in dichloromethane (3.5 mL), and the mixture was stirred at room temperature overnight. The solvent was evaporated under reduced pressure, and the residue was diluted with ethyl acetate. The mixture was washed with 0.1N hydrochloric acid, saturated aqueous sodium hydrogen carbonate solution and saturated brine, dried over anhydrous magnesium sulfate, and concentrated under reduced pressure to give the title compound without purification.
yield (390 mg, 0.705 mmol, quantitative)

### step 2 Synthesis of 2-[amino(imino)methyl]-1,2,3,4-tetrahydroisoquinolin-7-yl 4-({1-[amino(imino)methyl]piperidin-4-yl}oxy)benzoate ditrifluoroacetate

To a solution (1.5 mL) of tert-butyl 7-[(4-{[1-(tert-butoxycarbonyl)piperidin-4-yl]oxy}benzoyl)oxy]-3,4-dihydroisoquinoline-2(1H)-carboxylate (189 mg, 0.342 mmol) in 1,4-dioxane was added 4 N hydrochloric acid/1,4-dioxane solution (6 mL), and the mixture was stirred at room temperature for 3 hr. The solvent was evaporated under reduced pressure, and the obtained residue was dissolved in DMF (3 mL). 1H-Pyrazole-1-carboxamidine hydrochloride (122 mg, 0.832 mmol) and diisopropylethylamine (0.381 mL, 2.19 mmol) were added, and the mixture was stirred at room temperature overnight. 1H-Pyrazole-1-carboxamidine hydrochloride (50 mg, 0.342 mmol) and diisopropylethylamine (0.120 mL, 0.684 mmol) were added, and the mixture was stirred at room temperature for 4 hr. 1H-Pyrazole-1-carboxamidine hydrochloride (100 mg, 0.684 mmol) and diisopropylethylamine (0.120 mL, 0.684 mmol) were added, and the mixture was stirred at room temperature for 2 hr, and successively stirred at 40°C for 0.5 hr. The mixture was concentrated under reduced pressure, and the obtained residue was purified by reversed-phase HPLC in the same manner as in Example 1, step 3, to give the title compound.
yield (86 mg, 0.129 mmol, 43%)
MS (ESI, m/z) 219 [M+H]²⁺
¹H-NMR (DMSO-d₆)δ1.66-1.74 (m, 2H), 2.02-2.11 (m, 2H), 2.93-2.96 (m, 2H), 3.34-3.45 (m, 2H), 3.62-3.72 (m, 4H), 4.60 (s, 2H), 4.81-4.88 (m, 1H), 7.06-7.21 (m, 4H), 7.34 (d, 1H), 7.46 (s, 4H), 7.52 (s, 4H), 8.07 (d, 2H).

### Example 13 2-(1-iminoethyl)-1,2,3,4-tetrahydroisoquinolin-7-yl 4-{[1-(1-iminoethyl)piperidin-4-yl]oxy}benzoate ditrifluoroacetate

To a solution (1.5 mL) of tert-butyl 7-[(4-{[1-(tert-butoxycarbonyl)piperidin-4-yl]oxy}benzoyl)oxy]-3,4-dihydroisoquinoline-2(1H)-carboxylate (201 mg, 0.363 mmol) in 1,4-dioxane was added a solution (6 mL) of 4 N hydrochloric acid/1,4-dioxane, and the mixture was stirred at room temperature for 3 hr. The solvent was evaporated under reduced pressure, and the obtained residue was dissolved in dehydrated ethanol (3 mL). Ethyl acetimidate hydrochloride (108 mg, 0.874 mmol) and diisopropylethylamine (0.405 mL, 2.32 mmol) were added, and the mixture was stirred at room temperature overnight and concentrated under reduced pressure. The obtained residue was purified by reversed-phase HPLC in the same manner as in Example 1, step 3, to give the title compound.
yield (132 mg, 0.199 mmol, 62%)
MS (ESI, m/z) 218 [M+H]²⁺
¹H-NMR (DMSO-d₆)δ1.75-1.88 (m, 2H), 2.07-2.18 (m, 2H), 2.30 (s, 3H), 2.38 (d, 3H), 3.00 (q, 2H), 3.52-3.60 (m, 2H), 3.68 (t, 2H), 3.75-3.82 (m, 2H), 4.73 (d, 2H), 4.86-4.93 (m, 1H), 7.06-7.22 (m, 4H), 7.37 (dd, 1H), 8.08 (dd, 2H), 8.63 (s, 1H), 8.67 (d, 1H), 9.17 (s, 1H), 9.26 (d, 1H).

### Example 14 2-[amino(imino)methyl]-1,2,3,4-tetrahydroisoquinolin-7-yl 4-{[1-(1-iminoethyl)piperidin-4-yl]oxy}benzoate ditrifluoroacetate

### step 1 Synthesis of benzyl 4-{4-[({2-[amino(imino)methyl]-1,2,3,4-tetrahydroisoquinolin-7-yl}oxy)carbonyl]phenoxy}piperidine-1-carboxylate trifluoroacetate

4-({1-[(Benzyloxy)carbonyl]piperidin-4-yl}oxy)benzoic acid (170 mg, 0.478 mmol), tert-butyl 7-hydroxy-3,4-dihydroisoquinoline-2(1H)-carboxylate (119 mg, 0.478 mmol), DCC (109 mg, 0.526 mmol) and DMAP (87.6 mg, 0.717 mmol) were mixed in dichloromethane (2.5 mL), and the mixture was stirred at room temperature overnight. The insoluble material was filtered off, and the filtrate was concentrated under reduced pressure. The residue was diluted with ethyl acetate, and washed with saturated aqueous sodium hydrogen carbonate solution and saturated aqueous ammonium chloride solution. The mixture was dried over anhydrous magnesium sulfate, and concentrated under reduced pressure. The obtained residue was dissolved in 1,4-dioxane (2.5 mL), 4 N hydrochloric acid/1,4-dioxane solution (7.5 mL) was added, and the mixture was stirred at room temperature for 4.5 hr. The solvent was evaporated under reduced pressure, DMF (5 mL), 1H-pyrazole-1-carboxamidine hydrochloride (84.3 mg, 0.575 mmol) and diisopropylethylamine (0.250 mL, 6.26 mmol) were added, and the mixture was stirred at room temperature overnight. 1H-Pyrazole-1-carboxamidine hydrochloride (35 mg, 0.240 mmol) and diisopropylethylamine (0.042 mL, 0.240 mmol) were added, and the mixture was stirred at room temperature for 1.5 hr. The solvent was evaporated under reduced pressure, and the obtained residue was purified by reversed-phase HPLC in the same manner as in Example 1, step 3, to give the title compound.
yield (170 mg, 0.265 mmol, 55%)
MS (ESI, m/z) 529 [M+H]⁺
¹H-NMR (DMSO-d₆)δ1.55-1.67 (m, 2H), 1.94-2.03 (m, 2H), 2.94 (t, 2H), 3.28-3.37 (m, 2H), 3.63 (t, 2H), 3.71-3.79 (m, 2H), 4.60 (s, 2H), 4.74-4.82 (m, 1H), 5.10 (s, 2H), 7.06-7.19 (m, 4H), 7.31-7.39 (m, 6H), 7.46 (br s, 4H), 8.06 (d, 2H).

### step 2 Synthesis of 2-[amino(imino)methyl]-1,2,3,4-tetrahydroisoquinolin-7-yl 4-{[1-(1-iminoethyl)piperidin-4-yl]oxy}benzoate ditrifluoroacetate

To a solution (5 mL) of benzyl 4-{4-[({2-[amino(imino)methyl]-1,2,3,4-tetrahydroisoquinolin-7-yl}oxy)carbonyl]phenoxy}piperidine-1-carboxylate trifluoroacetate (166 mg, 0.258 mmol) in acetic acid was added 10% palladium/carbon (20 mg), and the mixture was stirred at room temperature for 2 hr under a hydrogen atmosphere. The insoluble material was filtered off, and the solvent was evaporated under reduced pressure. To the obtained residue were added ethyl acetimidate hydrochloride (38.3 mg, 0.310 mmol), dehydrated ethanol (2.5 mL), DMF (2.5 mL) and diisopropylethylamine (0.225 mL, 1.29 mmol), and the mixture was stirred at room temperature for 4 hr. The mixture was concentrated under reduced pressure, and the obtained residue was purified by reversed-phase HPLC in the same manner as in Example 1, step 3, to give the title compound.
yield (109 mg, 0.164 mmol, 64%)
MS (ESI, m/z) 436 [M+H]⁺
¹H-NMR (DMSO-d₆)δ1.73-1.88 (m, 2H), 2.06-2.18 (m, 2H), 2.30 (s, 3H), 2.94 (t, 2H), 3.51-3.66 (m, 4H), 3.73-3.85 (m, 2H), 4.60 (s, 2H), 4.86-4.94 (m, 1H), 7.06 (d, 1H), 7.14 (dd, 1H), 7.20 (d, 2H), 7.34 (d, 1H), 7.52 (s, 4H), 8.08 (d, 2H), 8.63 (s, 1H), 9.18 (s, 1H).

### Example 15 4-{[1-(1-iminoethyl)piperidin-4-yl]oxy}benzyl 2-[amino(imino)methyl]-1,2,3,4-tetrahydroisoquinoline-7-carboxylate ditrifluoroacetate

### step 1 Synthesis of benzyl 4-[4-(hydroxymethyl)phenoxy]piperidine-1-carboxylate

An operation similar to that of Example 2, step 2, was performed using benzyl 4-[4-(methoxycarbonyl)phenoxy]piperidine-1-carboxylate (719 mg, 1.95 mmol) as a starting material to give the title compound without purification.
yield (455 mg, 1.33 mmol, 68%)
¹H-NMR (DMSO-d₆)δ1.72-1.84 (m, 2H), 1.86-1.98 (m, 2H), 3.42-3.51 (m, 2H), 3.70-3.79 (m, 2H), 4.49 (sept, 1H), 4.62 (d, 2H), 5.14 (s, 2H), 6.87-6.92 (m, 2H), 7.26-7.37 (m, 7H).

### step 2 Synthesis of 4-({1-[(benzyloxy)carbonyl]piperidin-4-yl}oxy)benzyl 2-[amino(imino)methyl]-1,2,3,4-tetrahydroisoquinoline-7-carboxylate trifluoroacetate

An operation similar to that of Example 14, step 1, was performed using benzyl 4-[4-(hydroxymethyl)phenoxy]piperidine-1-carboxylate (90 mg, 0.263 mmol) and 2-(tert-butoxycarbonyl)-1,2,3,4-tetrahydroisoquinoline-7-carboxylic acid (73 mg, 0.263 mmol) as starting materials to give the title compound.
yield (47 mg, 0.0716 mmol, 27%)
MS (ESI, m/z) 543 [M+H]⁺

### step 3 Synthesis of 4-{[1-(1-iminoethyl)piperidin-4-yl]oxy}benzyl 2-[amino(imino)methyl]-1,2,3,4-tetrahydroisoquinoline-7-carboxylate ditrifluoroacetate

An operation similar to that of Example 14, step 2, was performed using 4-({1-[(benzyloxy)carbonyl]piperidin-4-yl}oxy)benzyl 2-[amino(imino)methyl]-1,2,3,4-tetrahydroisoquinoline-7-carboxylate trifluoroacetate (61 mg, 0.0929 mmol) as a starting material to give the title compound.
yield (42 mg, 0.0615 mmol, 66%)
MS (ESI, m/z) 450 [M+H]⁺
¹H-NMR (DMSO-d₆)δ1.68-1.84 (m, 2H), 2.00-2.12 (m, 2H), 2.29 (s, 3H), 2.99 (t, 2H), 3.48-3.58 (m, 2H), 3.63 (t, 2H), 3.69-3.81 (m, 2H), 4.65 (s, 2H), 4.68-4.77 (m, 1H), 5.28 (s, 2H), 7.04 (d, 2H), 7.39-7.43 (m, 3H), 7.48 (s, 4H), 7.75 (m, 1H), 7.82-7.85 (m, 1H), 8.59 (br s, 1H), 9.13 (br s, 1H).

### Example 16 {2-[amino(imino)methyl]-1,2,3,4-tetrahydroisoquinolin-7-yl}methyl 4-{[1-(1-iminoethyl)piperidin-4-yl]oxy}benzoate ditrifluoroacetate

### step 1 Synthesis of benzyl 4-{4-[({2-[amino(imino)methyl]-1,2,3,4-tetrahydroisoquinolin-7-yl}methoxy)carbonyl]phenoxy}piperidine-1-carboxylate trifluoroacetate

An operation similar to that of Example 14, step 1, was performed using 4-({1-[(benzyloxy)carbonyl]piperidin-4-yl}oxy)benzoic acid (181 mg, 0.509 mmol) and tert-butyl 7-(hydroxymethyl)-3,4-dihydroisoquinoline-2(1H)-carboxylate (134 mg, 0.509 mmol) as starting materials to give the title compound.
yield (229 mg, 0.349 mmol, 68%)
MS (ESI, m/z) 543 [M+H]⁺
¹H-NMR (DMSO-d₆)δ1.52-1.65 (m, 2H), 1.90-2.01 (m, 2H), 2.91 (t, 2H), 3.24-3.37 (m, 2H), 3.60 (t, 2H), 3.67-3.78 (m, 2H), 4.58 (s, 2H), 4.68-4.77 (m, 1H), 5.09 (s, 2H), 5.30 (s, 2H), 7.09 (d, 2H), 7.22-7.36 (m, 8H), 7.43 (s, 4H), 7.92 (d, 2H).

### step 2 Synthesis of {2-[amino(imino)methyl]-1,2,3,4-tetrahydroisoquinolin-7-yl}methyl 4-{[1-(1-iminoethyl)piperidin-4-yl]oxy}benzoate ditrifluoroacetate

An operation similar to that of Example 14, step 2, was performed using benzyl 4-{4-[({2-[amino(imino)methyl]-1,2,3,4-tetrahydroisoquinolin-7-yl}methoxy)carbonyl]phenoxy}piperidine-1-carboxylate trifluoroacetate (225 mg, 0.343 mmol) as a starting material to give the title compound.
yield (34.3 mg, 0.0506 mmol, 15%)
MS (ESI, m/z) 450 [M+H]⁺
¹H-NMR (DMSO-d₆)δ 1.71-1.86 (m, 2H), 2.03-2.16 (m, 2H), 2.29 (s, 3H), 2.92 (t, 2H), 3.48-3.64 (m, 4H), 4.58 (s, 2H), 4.80-4.88 (m, 1H), 5.31 (s, 2H), 7.12 (d, 2H), 7.23-7.35 (m, 3H), 7.49 (s, 4H), 7.94 (d, 2H), 8.61 (s, 1H), 9.16 (s, 1H).

### Example 17 {5-[amino(imino)methyl]-1-[2-(cyclohexylamino)-2-oxoethyl]-1H-benzimidazol-2-yl}methyl 1-(pyridin-4-yl)piperidine-4-carboxylate ditrifluoroacetate

### step 1 Synthesis of tert-butyl N-(2-{[(benzyloxy)acetyl]amino}-4-cyanophenyl)glycinate

2-Benzyloxyacetic acid (0.10 mL, 0.72 mmol), HOAt (117 mg, 0.86 mmol) and HATU (330 mg, 0.86 mmol) were dissolved in DMF (1 mL) and triethylamine (0.15 mL, 1.1 mmol), and the mixture was stirred for 5 min. Thereto was added tert-butyl N-(2-amino-4-cyanophenyl)glycinate (178 mg, 0.72 mmol), and the mixture was stirred overnight. After treatment with ethyl acetate and 1N aqueous sodium hydroxide solution, the mixture was washed with saturated brine, and dried over anhydrous sodium sulfate. The solvent was evaporated, and the residue was purified by reversed-phase HPLC in the same manner as in Example 1, step 3, to give the title compound.
yield (180 mg, 61%)
MS (ESI, m/z) 396 [M+H]⁺

### step 2 Synthesis of {5-[amino(imino)methyl]-1-[2-(cyclohexylamino)-2-oxoethyl]-1H-benzimidazol-2-yl}methyl 1-(pyridin-4-yl)piperidine-4-carboxylate ditrifluoroacetate

tert-Butyl N-(2-{[(benzyloxy)acetyl]amino}-4-cyanophenyl)glycinate (0.12 g, 0.37 mmol) was dissolved in acetic acid (10 mL), and the mixture was stirred at 90°C overnight. After evaporation of the solvent, trifluoroacetic acid (5 mL) was added, and the mixture was stirred for 2 hr. After evaporation of the solvent, water was added and the mixture was freeze-dried to give an intermediate. The obtained intermediate, HOAt (61 mg, 0.45 mmol) and HATU (170 mg, 0.45 mmol) were dissolved in DMF (2 mL) and triethylamine (0.1 mL, 0.73 mmol), and the mixture was stirred for 5 min. Thereto was added cyclohexylamine (0.043 mL, 0.37 mmol), and the mixture was stirred overnight. After working up according to a conventional method, the solvent was evaporated. The obtained compound was dissolved in 4 N hydrochloric acid/1,4-dioxane solution (4.5 mL) and ethanol (0.5 mL), and the mixture was stirred overnight. After evaporation of the solvent, ethanol (5 mL) and ammonium carbonate (0.12 g, 1.25 mmol) were added, and the mixture was stirred overnight. The insoluble material was filtered off, and the solvent was evaporated. The obtained compound was dissolved in ethanol (10 mL). 10% Pd/carbon in a catalytic amount was added, and the mixture was stirred overnight under a hydrogen atmosphere. The catalyst was filtered off, and the solvent was evaporated to give an intermediate.
Dichloromethane (1 mL), DMF (0.5 mL) and phosphorus oxychloride (0.005 mL, 0.05 mmol) were mixed at 0°C, and the mixture was stirred for 10 min. Thereto was added 1-(pyridin-4-yl)piperidine-4-carboxylic acid (9 mg, 0.045 mmol), and the mixture was stirred for 10 min. Thereto was added a solution (1 mL) of the above-mentioned intermediate in DMF, and the mixture was stirred overnight. The solvent was evaporated, and the residue was purified by reversed-phase HPLC in the same manner as in Example 1, step 3, to give the title compound.
yield (2.0 mg, 6%)
MS (ESI, m/z) 518 [M+H]⁺

Table 1 shows structural formulas of the compounds described in the Examples, wherein TFA is trifluoroacetate.

**Table 1**

| | | | |
|---|---|---|---|
| compound of Ex. 1 | 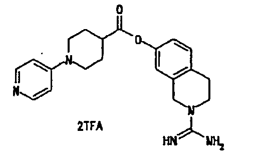 | compound of Ex. 10 | 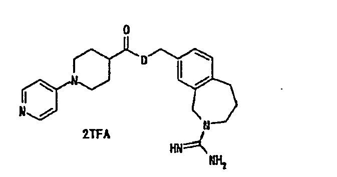 |
| compound of Ex. 2 | 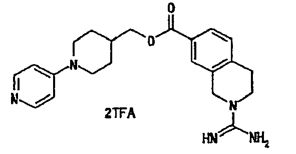 | compound of Ex. 11 | 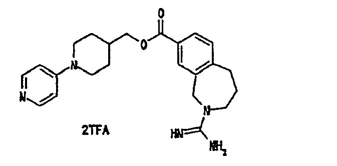 |
| compound of Ex. 3 | 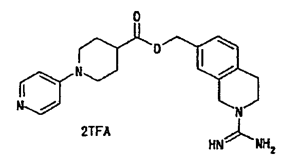 | compound of Ex. 12 | 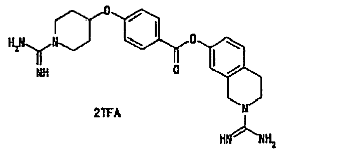 |
| compound of Ex. 4 | 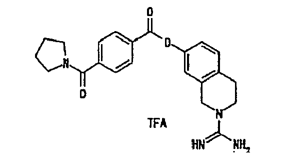 | compound of Ex. 13 | 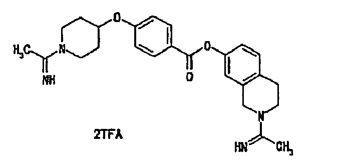 |
| compound of Ex. 5 | 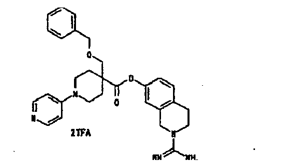 | compound of Ex. 14 | 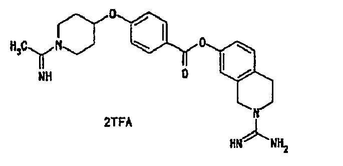 |
| compound of Ex. 6 | 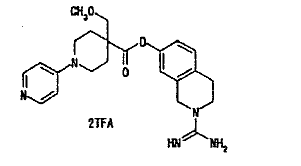 | compound of Ex. 15 | 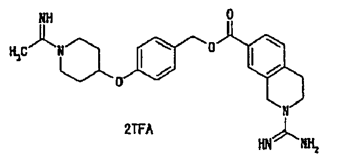 |
| compound of Ex. 7 | 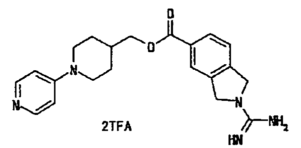 | compound of Ex. 16 | 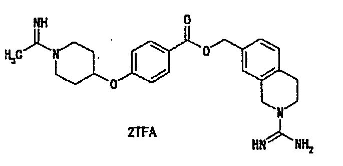 |
| compound of Ex. 8 | 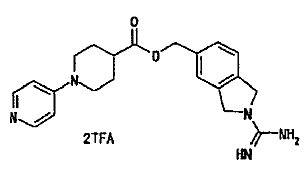 | compound of Ex. 17 | 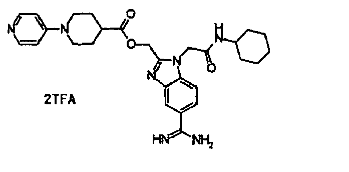 |
| compound of Ex. 9 | 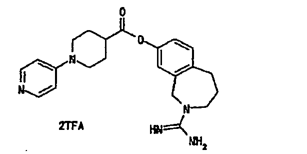 | | |

### Experimental Example 1 Measurement of activated factor X activity inhibitory activity

Using a 96 well plate (#3396, Costar), Tris-HCl buffer (100 mM, 130 µL) containing 0.02% Tween 20, 0.1% PEG6000 and 0.2 M NaCl was blended with FXa (0.015 U/ml, 10 µL) and a test compound (10 µL) for 10 min, and a chromogenic substrate (0.2 mM, S-2222, 50 µL) was added. Using microplate reader Benchmark Plus (BIO-RAD), the reaction rate was measured from the time course changes in the absorbance at 405 nm. The reaction rate of the control was taken as 100%, and a negative logarithmic value of a concentration at which the reaction rate of the control could be suppressed to 50% was taken as the pIC₅₀ value. The results are shown by pIC₅₀ (FXa) in Table 2.

### Experimental Example 2 Measurement of activated factor II (FIIa, thrombin)-inhibitory activity

Using a 96 well plate (#3396, Costar), Tris-HCl buffer (100 mM, 130 µL) containing 0.02% Tween 20, 0.1% PEG6000 and 0.2 M NaCl was blended with activated factor IIa (thrombin, 0.125 U/mL, 10 µL) and a test compound (10 µL) for 10 min, and a chromogenic substrate (0.1 mM, S-2238, 50 µL) was added. Using microplate reader Benchmark Plus (BIO-RAD), the reaction rate was measured from the time course changes of absorbance at 405 nm. The reaction rate of the control was taken as 100%, and a negative logarithmic value of a concentration at which the reaction rate of the control could be suppressed to 50% was taken as the pIC₅₀ value. The results are shown by pIC₅₀ (IIa) in Table 2.

### Experimental Example 3 Measurement of anti-blood coagulant activity

The test was performed according to an aPTT measurement method using a fully-automated blood coagulation time measuring apparatus Sysmex CA-1500. In a sample tube (MS-18, Japan Medical Science) were placed DDVP solution (10 mg/ml, DDVP standard product, Wako Corporation, 4 µL), a solution (20 µL) of a test compound, and human plasma (standard human plasma for blood coagulation test, GCH-100A, Sysmex Corporation, 180 µL) and this was used as a test sample. The test sample (50 µL) was incubated at 37°C for 1 min, dataphay. APTT (cephalin derived from rabbit brain, DADE Behiring, 50 µL) was added, and the mixture was further incubated at 37°C for 2 min. To the sample solution was added calcium chloride (0.02 M, 50 µL), and the time before coagulation of the plasma was automatically measured.
The anti-blood coagulation activity was shown by the negative logarithmic value of a concentration at which aPTT of the control was double-extended as paPTT2. The results are shown in Table 2.

### Experimental Example 4 Evaluation of stability in plasma

To human plasma (495 µL) was added a solution (5 µL) of the test compound prepared to 100 µg/mL (final drug solution concentration 1 µg/mL), and the mixture was incubated at 37°C. At 0 min, 2 min, 5 min, 10 min, 30 min and 60 min after the addition of the drug solution, 50 µL each was sampled. Acetonitrile (100 µL) was added and the mixture was admixed to discontinue the reaction. After discontinuation of the reaction, a protein elimination treatment was performed by centrifugation (15000 rpm, 5 min). The centrifugation supernatant (20 µL) was diluted with ammonium formate solution (100 mmol/L, pH 4, 180 µL), and subjected to the measurement by LC/MS/MS.
When the value at 2 min after the addition of the drug solution was less than the quantification limit, the half-life was calculated by the following method.
That is, the value at 0 min was taken as the theoretical concentration (1000 ng/mL) and the value at 2 min was taken as the quantitative lower limit concentration. The half-life (T_{1/2}) was calculated from the inclination of the straight line connecting the logarithmic conversion values at the two points. The results are shown in Table 2.

**Table 2**

| | pIC₅₀ (FXa) | pIC₅₀ (IIa) | paPTT2 | T_{1/2} (sec) |
|---|---|---|---|---|
| compound of Ex. 2 | 6.4 | <4 | 5.4 | <23.7 |
| compound of Ex. 3 | 7.1 | 4.2 | 6.1 | <23.9 |
| compound of Ex. 5 | 6.3 | 4.7 | 5.4 | |
| compound of Ex. 6 | 5.8 | 4.4 | 5.4 | |
| compound of Ex. 7 | 5.1 | <4 | 4.6 | |
| compound of Ex. 8 | 5.5 | <4 | <5 | |
| compound of Ex. 15 | 5.0 | <4 | 4.4 | |
| compound of Ex. 16 | 5.3 | <4 | 4.5 | |

### Industrial Applicability

As shown in the aforementioned Experimental Examples, the compounds represented by the formulas (1) and (5), and pharmaceutically acceptable salts thereof have a high FXa inhibitory activity and a high anti(blood)coagulation action, and can be utilized as an anti(blood)coagulation drug(agent), as a therapeutic or prophylactic drug for various diseases in which Fxa-dependent coagulation process is pathologically involved, for example, thrombus formation in extracorporeal blood circulation, cerebral infarction, cerebral thrombus, cerebral embolism, transient cerebral ischemic attack (TIA), acute and chronic myocardial infarction, unstable angina pectoris, pulmonary obliteration, peripheral arterial obstruction, deep vein thrombosis, disseminated intravascular coagulation syndrome, thrombus formation after artificial blood vessel operation and artificial valve replacement, reocclusion and restenosis after coronary-artery bypass surgery, reocclusion and restenosis after blood vessel reconstruction such as percutaneous transluminal coronary angioplasty (PTCA), percutaneous transluminal coronary recanalization (PTCR) and the like.

Particularly, a compound represented by the formula (1) or (5), or a pharmaceutically acceptable salt thereof is useful as an anti(blood)coagulation drug(agent) for a circuit for extracorporeal blood circulation (e.g., hemodialyzer, artificial heart lung apparatus etc.).

In addition, a compound represented by the formula (1) or (5), or a pharmaceutically acceptable salt thereof is rapidly cleared from the blood. That is, since it has a short half-life in blood, hemostasis is easy when a bleeding symptom is observed on administration, and it is useful as an anti(blood)coagulation drug(agent) which can be used safely.

Furthermore, a compound represented by the formula (1) or (5), or a pharmaceutically acceptable salt thereof has a low thrombin-inhibitory activity, is an FXa selective inhibitor, and is an anti(blood)coagulation drug(agent) which can be used safely from the viewpoint of bleeding risk.

In addition, the low molecular weight FXa inhibitor, for example, a compound represented by the formula (1) or (5), or a pharmaceutically acceptable salt thereof is useful as an anti(blood)coagulation drug(agent) to be used for extracorporeal blood circulation/circuit for extracorporeal blood circulation.

Particularly, a selective low molecular weight FXa inhibitor with a short half-life in blood, which is rapidly cleared from the blood, for example, a compound represented by the formula (1) or (5) can be used safely and conveniently as an anti(blood)coagulation drug(agent) for prevention of blood coagulation in a circuit for extracorporeal blood circulation, and is useful since a treatment of and attention to hemostasis after the completion of extracorporeal blood circulation can be evidently reduced.

In addition, the present invention can also provide a method of preventing thrombus formation in a circuit for extracorporeal blood circulation, which comprises incorporating a low molecular weight FXa inhibitor into a constituent element of a circuit for extracorporeal blood circulation.

This application is based on patent application No. 2008-181882 filed in Japan, the contents of which are incorporated in full herein.

## Claims

1. An amidine derivative represented by the following formula (1): 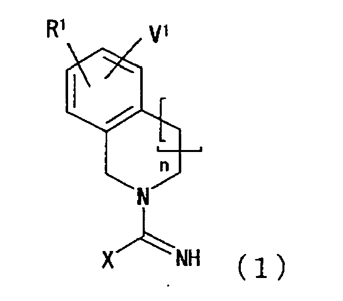 wherein,
X is a C₁₋₆ alkyl group or an amino group,
V¹ is a hydrogen atom, a hydroxyl group, a halogen atom,
a C₁₋₁₀ alkyl group optionally having substituent (s) , a C₁₋₁₀ alkoxy group optionally having substituent(s), a C₁₋₁₀ alkylamino group optionally having substituent(s), an aliphatic nitrogen-containing heterocyclic group having 2 to 8 carbon atoms, a C₁₋₁₀ alkylthio group optionally having substituent(s), a cyano group, a nitro group, a carboxyl group, a carbamoyl group optionally having substituent(s) or a C₂₋₁₀ alkoxycarbonyl group optionally having substituent(s),
n is an integer of 0 to 2, and
R¹ is a group represented by the following formula (2-1) or (2-2): 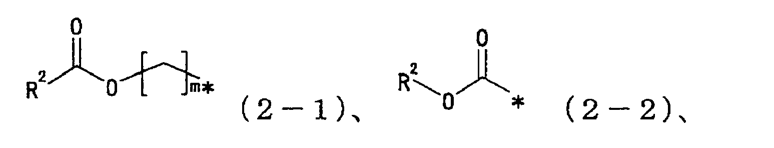 wherein,
m is an integer of 0 to 2, and
R² is a group represented by the following formula (3): 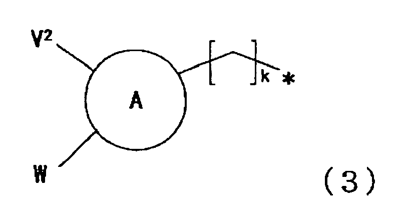 wherein,
k is an integer of 0 to 2,
ring A is a C₆₋₁₀ aryl group, a C₁₋₁₀ heteroaryl group, an aliphatic nitrogen-containing heterocyclic group having 2 to 8 carbon atoms or a C₃₋₁₀ cycloalkyl group,
V² is a hydrogen atom, a hydroxyl group, a halogen atom,
a C₁₋₁₀ alkyl group optionally having substituent(s), a C₁₋₁₀ alkoxy group optionally having substituent(s), a C₁₋₁₀ alkylamino group optionally having substituent(s), an aliphatic nitrogen-containing heterocyclic group having 2 to 8 carbon atoms, a C₁₋₁₀ alkylthio group optionally having substituent(s), a cyano group, a nitro group, a carboxyl group, a carbamoyl group optionally having substituent(s) or a C₂₋₁₀ alkoxycarbonyl group optionally having substituent(s), and
W is an amidino group optionally substituted by C₁₋₆ alkyl group(s), a guanidino group optionally substituted by C₁₋₆ alkyl group(s), a C₁₋₆ alkyl group optionally having an imino group at the 1-position or a group represented by the following formula (4) : 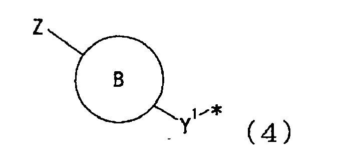 wherein,
ring B is a C₁₋₁₀ heteroaryl group, or an aliphatic nitrogen-containing heterocyclic group having 2 to 8 carbon atoms,
Y¹ is a single bond, -NH- optionally substituted by a C₁₋₆ alkyl group, an oxygen atom, a sulfur atom, a methylene group or -CO-, and
Z is a hydrogen atom, a halogen atom, an amidino group optionally substituted by C₁₋₆ alkyl group(s), a guanidino group optionally substituted by C₁₋₆ alkyl group(s), or a C₁₋₆ alkyl group optionally having an imino group at the 1-position, or a pharmaceutically acceptable salt thereof.

2. The amidine derivative according to claim 1, which is represented by the following formula (1-2): 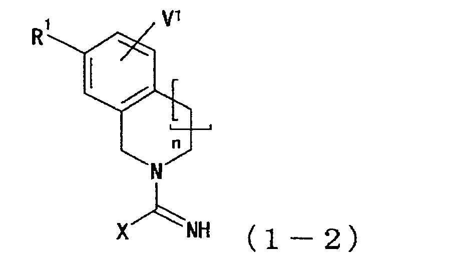 wherein R¹, V¹, X and n are as defined in claim 1,
or a pharmaceutically acceptable salt thereof.

3. The amidine derivative according to claim 2, wherein, in the formula (3),
ring A is a phenyl group, a pyridyl group, a thiophenyl group, a piperidyl group or a piperazinyl group, and
V² is a hydrogen atom, a halogen atom, a C₁₋₆ alkyl group, a carboxyl group, a C₁₋₆ alkoxy group optionally having substituent(s) or a C₂₋₁₀ alkoxycarbonyl group optionally having substituent(s),
or a pharmaceutically acceptable salt thereof.

4. The amidine derivative according to claim 3, wherein, in the formula (3),
W is a group represented by the formula (4),
ring B is an aliphatic nitrogen-containing heterocyclic group having 2 to 8 carbon atoms,
Y¹ is an oxygen atom, a sulfur atom or a methylene group, and
Z is a hydrogen atom, a halogen atom, an amidino group or a C₁₋₆ alkyl group optionally having an imino group at the 1-position, or a pharmaceutically acceptable salt thereof.

5. The amidine derivative according to claim 3, wherein, in the formula (3),
W is a group represented by the formula (4),
ring B is a pyridyl group, and
Y¹ is a single bond,
or a pharmaceutically acceptable salt thereof.

6. An amidine derivative represented by the following formula (5): 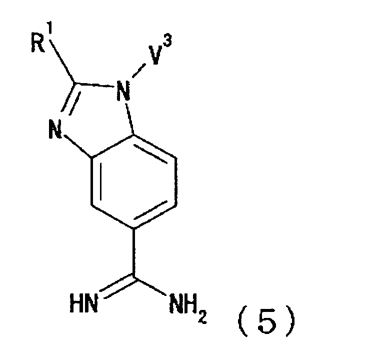 in the formula (5),
V³ is a hydrogen atom or a group represented by the following formula (6): 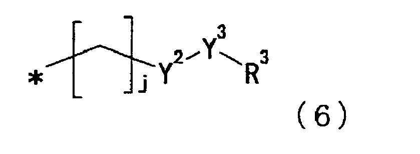 in the formula (6),
R³ is a hydrogen atom, a C₁₋₆ alkyl group optionally having substituent(s), a C₃₋₁₀ cycloalkyl group optionally having substituent(s), a carboxyl group, a C₂₋₇ alkoxycarbonyl group, a C₆₋₁₀ aryl group optionally having substituent(s), a heteroaryl group optionally having substituent(s) or a saturated aliphatic nitrogen-containing heterocyclic group having 2 to 8 carbon atoms,
Y² is an oxygen atom, -CO-, -CO₂- -SO₂- -CONH- or - CH=CH-,
Y³ is -(GH₂)ᵢ- or -(CH₂)_{i'}-CUU'-(CH₂)_{i"}- (wherein U and U' are the same or different and each is a hydrogen atom or a C₁₋₆ alkyl group, and i, i' and i" are each independently an integer of 0 to 3), and
j is an integer of 0 to 3,
R¹ is as defined in claim 1,
or a pharmaceutically acceptable salt thereof.

7. The amidine derivative according to claim 6, wherein, in the formula (5),
V³ is a group represented by the formula (6), and
in the formula (3),
ring A is a phenyl group, a pyridyl group, a thiophenyl group, a piperidyl group or a piperazinyl group, and
V² is a hydrogen atom, a halogen atom, a C₁₋₆ alkyl group, a carboxyl group, a C₁₋₆ alkoxy group optionally having substituent(s) or a C₂₋₁₀ alkoxycarbonyl group optionally having substituent(s),
or a pharmaceutically acceptable salt thereof.

8. The amidine derivative according to claim 7, wherein, in the formula (5),
V³ is a group represented by the formula (6), and
in the formula (3),
W is a group represented by the formula (4),
ring B is an aliphatic nitrogen-containing heterocyclic group having 2 to 8 carbon atoms,
Y¹ is an oxygen atom, a sulfur atom or a methylene group, and
Z is a hydrogen atom, an amidino group or a C₁₋₆ alkyl group optionally having an imino group at the 1-position,
or a pharmaceutically acceptable salt thereof.

9. The amidine derivative according to claim 7, wherein, in the formula (5),
V³ is a group represented by the formula (6), and
in the formula (3),
W is a group represented by the formula (4),
ring B is a pyridyl group, and
Y¹ is a single bond,
or a pharmaceutically acceptable salt thereof.

10. An activated blood coagulation factor X inhibitor comprising the amidine derivative according to any one of claims 1 to 9, or a pharmaceutically acceptable salt thereof.

11. A pharmaceutical composition comprising the amidine derivative according to any one of claims 1 to 9, or a pharmaceutically acceptable salt thereof.

12. The pharmaceutical composition according to claim 11, which is an anti-blood coagulation drug.

13. The pharmaceutical composition according to claim 12, which is an anti-blood coagulation drug for a circuit for extracorporeal blood circulation.

14. The pharmaceutical composition according to claim 12, which is an anti-blood coagulation drug for hemodialysis.

15. A dialysis solution or dialysis concentrate comprising the amidine derivative according to any one of claims 1 to 9, or a pharmaceutically acceptable salt thereof.

16. An anti-blood coagulation drug for a circuit for extracorporeal blood circulation, comprising a low molecular weight FXa inhibitor as an active ingredient.

17. The anti-blood coagulation drug according to claim 16, wherein the low molecular weight FXa inhibitor is rapidly cleared from the blood.

18. The anti-blood coagulation drug according to claim 17, wherein the low molecular weight FXa inhibitor is a selective FXa inhibitor.
